# EUROPÄISCHE PATENTSCHRIFT

(11) **EP 3 254 093 B1**
(45) Veröffentlichungstag und Bekanntmachung des Hinweises auf die Patenterteilung: **27.05.2020**
(21) Anmeldenummer: 16712199.5
(22) Anmeldetag: 02.02.2016
(51) Int. Cl.: G01N 27/04, G01N 33/15, A61J 1/03

(54) **VERFAHREN UND VORRICHTUNG ZUR BESTIMMUNG DES WASSERGEHALTS**
METHOD AND DEVICE FOR DETERMINING WATER CONTENT
PROCÉDÉ ET DISPOSITIF DE DÉTERMINATION D'UNE TENEUR EN EAU

(30) Priorität: 03.02.2015 EP 15000304
(43) Veröffentlichungstag der Anmeldung: 13.12.2017
(73) Patentinhaber: Boehringer Ingelheim International GmbH, 55216 Ingelheim am Rhein (DE)
(72) Erfinder: BALTHES, Eduard, 55216 Ingelheim am Rhein (DE); SALZMANN, Kathrin, 55216 Ingelheim am Rhein (DE)
(74) Vertreter: Von Rohr Patentanwälte Partnerschaft mbB
(86) Internationale Anmeldenummer: PCT/EP2016/000162
(87) Internationale Veröffentlichungsnummer: WO 2016/124326

(56) Entgegenhaltungen:
- EP-A1- 1 345 027
- DE-A1-102007 034 156
- JP-A- H 061 359
- JP-A- H10 225 501
- US-A- 4 657 133

## Beschreibung

Die vorliegende Erfindung betrifft ein Verfahren zur Bestimmung des Wassergehalts einer Probe in Form einer Arzneimittelzubereitung und/oder eines Verpackungsmaterials sowie eine Messkammer.

Die vorliegende Erfindung betrifft insbesondere den Bereich der Arzneimittel, insbesondere die Wahl und Konstruktion von Verpackungen für Arzneimittelzubereitungen.

Es hat sich gezeigt, dass sich der Wassergehalt einer Arzneimittelzubereitung und/oder eines Verpackungsmaterials maßgeblich auf die Haltbarkeit der Arzneimittelzubereitung auswirkt. Die Erfindung betrifft ferner die genaue Bestimmung des Wassergehalts einer Arzneimittelzubereitung/Probe und die Simulation von Haltbarkeiten der Arzneimittelzubereitung/Probe verpackt in der jeweiligen Verpackung, um eine besonders geeignete bzw. kostengünstige Verpackung zu ermitteln. Hierfür ist es besonders wünschenswert, den Wassergehalt einer Mehrzahl von Proben einer Arzneimittelzubereitung und/oder eines Verpackungsmaterials möglichst schnell und exakt zu bestimmen.

Bisher bekannt und üblich im Bereich der Arzneimittel sind Verfahren, bei denen durch einen Trocknungsvorgang Rückschlüsse auf den ursprünglichen Wassergehalt gezogen werden.

Zu solchen Verfahren zählen Differenzwägungen, bei denen eine feuchte Probe, insbesondere in Form der Arzneimittelzubereitung und/oder des Verpackungsmaterials, gewogen, danach unter Temperaturerhöhung getrocknet und daraufhin erneut gewogen werden. Alternativ oder zusätzlich können auch Trocken- bzw. Trocknungsmittel zum Trocknen der Proben, insbesondere in Form der Arzneimittelzubereitungen und/oder des Verpackungsmaterialien, eingesetzt werden. Aus der Gewichtsdifferenz wird dann auf einen ursprünglichen Wassergehalt der Probe, insbesondere in Form der Arzneimittelzubereitung und/oder des Verpackungsmaterials, geschlossen.

Bei der Karl-Fischer-Titration wird eine Probe in einem geeigneten Lösungsmittel gelöst und mit einer anschließenden Titration das in Lösung gegangene Wasser erfasst.

Bei der Calciumcarbid-Methode wird die Arzneimitteilzubereitung zusammen mit Calciumcarbid in einem druckdichten Behälter geben, in dem das Wasser aus der Probe mit dem Calciumcarbid unter Acetylenbildung reagiert, wodurch sich ein Überdruck bildet, der mit einem Manometer gemessen werden kann und zu der in der Probe enthaltenen Wassermenge korrespondiert.

Den zuvor genannten Verfahren ist gemein, dass sie zwar für viele Anwendungen ausreichend genau, jedoch sehr aufwendig und zeitintensiv sind.

Die EP 1 345 027 A1 betrifft eine Vorrichtung zur Bestimmung der Holzfeuchte in Holzstapeln mit Elektroden zur Messung eines elektrischen Widerstands. Der Widerstand zwischen den Elektroden wird vom feuchtesten Weg des Holzes bestimmt, das zwischen den Elektroden gelegen ist. Aus dem gemessenen Widerstand kann die Holzfeuchte bestimmt werden, da die Korrelation zwischen dem Widerstand und der Holzfeuchte für die meisten Holzarten bekannt ist.

Die JP H10 225501 A betrifft ein Verfahren zur Herstellung eines festen Arzneimittels. Hierbei wird für eine Beschichtung das Arzneimittel mit Wasser besprüht und der Feuchtegehalt durch Messung eines Widerstandswerts gesteuert.

Die DE 10 2007 034156 A1 betrifft eine Vorrichtung und ein Verfahren zur Bestimmung des Feuchtegehalts eines Verpackungsmaterials mittels der elektrischen Leitfähigkeit des Verpackungsmaterials.

Ein Ziel der vorliegenden Erfindung ist eine genaue, schnelle, einfache, zerstörungsfreie, breitbandig anwendbare Methode für Feuchtegehaltsmessungen zur Sicherstellung der Produktstabilität durch Anwendung in der Qualitätskontrolle (beispielsweise Wareneingang, Verarbeitung und Lagerung von Produktkomponenten, Zwischenprodukten, Bulkware, Packmittelkomponenten, Verpackungssystemen bzw. dem verpackten Produkt) sowie at-line, on-line oder sogar in-line von Produktions- bzw. Verpackungsprozessen.

Aus dem Bereich der Bestimmung von Restfeuchtigkeit in Holz oder Bauwerken sind Methoden bekannt, bei denen eine elektrische Leitfähigkeit bestimmt und auf dieser Basis ein Wassergehalt abgeschätzt wird. Die hierbei üblichen Toleranzen von mehreren Prozent sind zwar akzeptabel für eine Entscheidung, ob Holz trocken genug zum Verbrennen ist oder ein Mauerwerk einen Feuchtigkeitsschaden aufweist, nicht jedoch für den vorliegenden Bereich der Arzneimittel. Deshalb werden solche Verfahren im Bereich der Arzneimittel nicht eingesetzt.

Aufgabe der vorliegenden Erfindung ist es, ein Verfahren zur Bestimmung des Wassergehalts einer Probe in Form einer Arzneimittelzubereitung und/oder eines Verpackungsmaterials sowie eine Messkammer anzugeben, wodurch eine Bestimmung des Wassergehalts der Arzneimittelzubereitung und/oder des Verpackungsmaterials, schneller und/oder exakter durchführbar ist, insbesondere so dass auch die Feuchtigkeitsbestimmung von größeren Messreihen mit einer Vielzahl von Arzneimittelzubereitungs-Proben und/oder Verpackungsmaterial-Proben, handhabbar und/oder schneller realisierbar wird.

Diese Aufgabe wird durch ein Verfahren gemäß Anspruch 1 oder eine Messkammer gemäß Anspruch 7 gelöst. Vorteilhafte Weiterbildungen sind Gegenstand der Unteransprüche.

Im Folgenden wird die Erfindung allgemein bezogen auf "Proben" erläutert. Der beanspruchte Gegenstand der Erfindung betrifft jedoch nur Proben in Form von Arzneimittelzubereitungen und/oder Verpackungsmaterialien.

Es ist bevorzugt, dass es sich bei der/den Probe(n) um eine / mehrere Arzneimittelzubereitungen handelt. In diesem Zusammenhang haben sich einige im Folgenden beschriebenen Aspekte der vorliegenden Erfindung als besonders vorteilhaft erwiesen. Daher kann der Begriff "Probe" im Sinne der vorliegenden Erfindung bedarfsweise durch den Begriff "Arzneimittelzubereitung" ersetzt bzw. zu dem Begriff "Arzneimittelzubereitungs-Probe" ergänzt werden.

Alternativ oder zusätzlich ist die Probe durch ein Verpackungsmaterial gebildet. Folglich kann der im Folgenden verwendete Begriff "Probe" im Sinne der vorliegenden Erfindung durch den Begriff "Verpackungsmaterial" ersetzt oder zu dem Begriff "Verpackungsmaterial-Probe" ergänzt werden.

Insgesamt kann daher der Begriff "Probe" im Sinne der vorliegenden Erfindung stets auch durch die Kombination "Arzneimittelzubereitung und/oder Verpackungsmaterial" ersetzt werden.

Im Folgenden wird im Wesentlichen der Term "Arzneimittelzubereitung/Probe" verwendet, wobei dies "Arzneimittelzubereitung und/oder Probe" bedeutet und folglich durch nachträgliche Streichung einer Alternative auf "Probe" oder "Arzneimittelzubereitung" beschränkt werden kann. Ferner erfolgt die Nutzung von "Probe/Arzneimittelzubereitung" zum besseren Verständnis. Auch hier können die Terme "Probe" bzw. "Arzneimittelzubereitung" wie zuvor beschrieben ersetzt oder ergänzt werden.

Weiter wird die Erfindung anhand konkreter Ausführungsbeispiele betreffend eine oder mehrere Arzneimittelzubereitungen im Detail erläutert. Vorzugsweise ist die vorliegende Erfindung jedoch auch in diesem Zusammenhang nicht auf "Arzneimittelzubereitungen" beschränkt. Daher kann der Begriff "Arzneimittelzubereitungen" vorzugsweise durch den Begriff "Probe", den Begriff "Verpackungsmaterial" oder die Kombination "Arzneimittelzubereitung und/oder Verpackungsmaterial" ersetzt werden. Dies gilt jedenfalls soweit der jeweilige Zusammenhang einer Ersetzung nicht denklogisch entgegensteht.

Die vorliegende Erfindung betrifft vorrangig die Messung des Feuchtegehalts von Proben, vorzugsweise in Form von festen pharmazeutischen Formulierungen, deren Verpackungen, Packmittelkomponenten, insbesondere Trockenmitteln und Folien, sowie Devices für pharmazeutische Anwendungen mit Hilfe deren elektrischer Leitfähigkeit.

Unter dem Begriff "Probe" bzw. "Arzneimittelzubereitung" sind vorzugsweise alternativ oder zusätzlich zu verstehen: pharmazeutische Produkte (z.B. Tabletten, Pulver, Granulate, feste Formulierungen, Kapseln, Hilfsstoffe), Wirkstoffe, Rohstoffe, Vormischungen, Zwischenprodukte pharmazeutischer Zubereitungen, Kapseln, sowie Packmittel (Folien, Siegellacke, granulare Trockenmittel, Trockenmittel enthaltende Formteile, polymerbasierte Flaschen, polymerbasierte Devices, insbesondere für Inhalativa, für pädiatrische Anwendungen, für Anwendungen in der Tiergesundheit, für Diagnosezwecke entwickelte Devices), Verpackungen, Devices mit potenziellem Einsatz in der Entwicklung, und/oder einzelne Teile der genannten Komponenten.

Insbesondere kann die Bestimmung der elektrischen Leitfähigkeit als schnelle Methode für Voruntersuchungen hinsichtlich der Probenqualität von pharmazeutischen Wirkstoffen und/oder Hilfsstoffen genutzt werden. Hierzu wird vorzugsweise die Leitfähigkeit - vorzugsweise über deren Zusammenhänge mit der Feuchtigkeit oder dem Wassergehalt - einer Probenqualität zugeordnet. Hierdurch kann aus der ermittelten Leitfähigkeit - unmittelbar oder mittelbar - auf die Probenqualität rückgeschlossen werden. Auf diese Weise kann ein Qualitätsindikator korrespondierend zu der Probenqualität erzeugt, ermittelt und/oder ausgegeben werden. Der Qualitätsindikator kann ausschließlich die Feuchtigkeit bzw. den Wassergehalt der Probe oder zusätzlich auch einen oder mehrere andere qualitäts- oder haltbarkeitsbestimmende Parameter sein oder berücksichtigen.

Ein erster Aspekt der vorliegenden Erfindung betrifft ein Verfahren zur Bestimmung des Wassergehalts einer Probe in Form der Arzneimittelzubereitung und/oder des Verpackungsmaterials gemäß Anspruch 1, wobei eine Messkammer mit mindestens zwei Elektroden vorgesehen ist und die Arzneimittelzubereitung/Probe mit diesen Elektroden in unmittelbaren Kontakt gebracht wird. Dies erfolgt so, dass die Elektroden durch die Arzneimittelzubereitung/Probe miteinander verbunden werden, die Elektroden also lediglich über die Arzneimittelzubereitung/Probe und nicht direkt elektrisch miteinander in Kontakt stehen. Die Elektroden stehen dann über die Arzneimittelzubereitung/Probe miteinander in Kontakt. Weiter wird ein, insbesondere elektrischer und/oder ohmscher, Widerstand der Arzneimittelzubereitung/Probe mittels der Elektroden bestimmt und mit dem Widerstand wird der Wassergehalt der Arzneimittelzubereitung/Probe bestimmt.

Es hat sich in überraschender Weise gezeigt, dass entgegen der bisherigen Vorurteile eine Bestimmung des Wassergehalts einer Arzneimittelzubereitung/Probe durch Bestimmung des Widerstands dieser nicht nur sehr schnell ist, sondern auch sehr exakt sein kann. Hierzu trägt maßgeblich die Verwendung einer Messkammer bei, in der die Elektroden und die Arzneimittelzubereitung/Probe angeordnet sind. In der Messkammer können nämlich eine definierte Umgebung einschließlich einer vorgegebenen, die Arzneimittelzubereitung/Probe umgebenden Atmosphäre, und reproduzierbare Übergänge zwischen den Elektroden und der Arzneimittelzubereitung/Probe erreicht werden, was in synergistischer Weise zu einer wesentliche exakteren Bestimmung des Wassergehalts im Vergleich zu bekannten widerstandsbasierten Methoden führt, als zu erwarten war.

Die Erfindung ermöglicht eine genaue und/oder schnelle und/oder einfach durchführbare und/oder zerstörungsfreie (ohne Zerstörung der Probe bzw. der Form, Darreichungsform, Oberfläche oder Umfangslinie der Probe) und/oder breitbandig bzw. vielseitig anwendbare Methode für Feuchtegehaltsmessungen, mit Hilfe der elektrischen Leitfähigkeit.

Weiter hat sich in überraschender Weise gezeigt, dass mit der vorliegenden Erfindung eine genauere Bestimmung der Feuchtigkeit bzw. des Wassergehalts der Arzneimittelzubereitung/Probe ermöglicht wird. Durch die gesteigerte Genauigkeit ist es möglich, Sicherheitsreserven bzw. Sicherheitsaufschläge zu reduzieren. Beispielsweise ist es durch die genauere Bestimmung der Feuchtigkeit bzw. des Wassergehalts der Arzneimittelzubereitung/Probe möglich, die Menge des innerhalb einer Verpackung vorzusehenden Trocknungsmittels gering zu halten und folglich eine Ressourcenschonende Verpackung zu ermöglichen.

Es ist bevorzugt, dass zur Bestimmung des Wassergehalts ein Zuordnungsmittel verwendet wird, insbesondere eine Zuordnungstabelle oder Zuordnungsfunktion. Vorzugsweise wird oder ist durch das Zuordnungsmittel jeweils eine mit einer Referenz-Messmethode bestimmter Wassergehalt der Arzneimittelzubereitung/Probe ein bei demselben Wassergehalt der Arzneimittelzubereitung/Probe ermittelter Wischreibungseinleitung genannten Verfahren, bei denen durch einen Trocknungsvorgang präzise Rückschlüsse auf den ursprünglichen Wassergehalt gezogen werden können.

Es ist insbesondere vorgehsehen, dass eine Zuordnungstabelle oder Zuordnungsfunktion bzw. ein sonstiges Zuordnungsmittel spezifisch für eine ganz bestimmte Arzneimittelzubereitung/Probe, bevorzugt bei unterschiedlichen Wassergehalten ist oder hierfür bestimmt wird.

Für unterschiedliche Arzneimittelzubereitung/Proben, insbesondere also Arzneimittelzubereitungen/Proben mit unterschiedlichen Formulierungen oder Zusammensetzungen, können mehrere und/oder unterschiedliche, korrespondierende Zuordnungsmittel bestimmt, vorgesehen und/oder verwendet werden.

Es hat sich in überraschender Weise gezeigt, dass auch bei ähnlichen Formulierungen von Arzneimittelzubereitungen/Proben die vom Wassergehalt abhängigen Widerstände signifikant voneinander abweichen können und folglich individuelle Zuordnungsmittel der Genauigkeit der Bestimmung des Wassergehalts zuträglich sind.

Mit dem Zuordnungsmittel wird vorzugsweise ein zu dem Widerstand korrespondierender Wassergehalt bestimmt, insbesondere indem ein zu dem jeweils bestimmten Widerstand korrespondierender Wert aus der Zuordnungstabelle entnommen, mit der Zuordnungsfunktion berechnet oder mit dem sonstigen Zuordnungsmittel bestimmt wird. Der Wassergehalt oder eine hieraus ermittelte Haltbarkeit der Arzneimittelzubereitung/Probe wird/werden vorzugsweise als Ergebnis ausgegeben. Dies ermöglicht eine schnelle und genaue Bestimmung des Wassergehalts bzw. einer besonders vorteilhaften Verpackung, da unter Verwendung genauer Wassergehalte ggfs. die Menge an benötigten Trocknungsmittel in einer Verpackung oder die Menge an Verpackungsmaterial reduziert oder geändert oder die Komplexität der Verpackung reduziert werden kann.

Besonders bevorzugt ist das Zuordnungsmittel alternativ oder zusätzlich spezifisch für bestimmte Elektroden, oder umgekehrt. Es kann also vorgesehen sein, dass für dieselbe Arzneimittelzubereitung/Probe unterschiedliche Zuordnungsmittel für die Verwendung unterschiedlicher Elektroden vorgesehen sind oder verwendet, insbesondere (automatisch) ausgewählt, werden. Hierdurch kann vermieden werden, dass Unterschiede in den Kontaktflächen zwischen den Elektroden und der Arzneimittelzubereitung/Probe zu Abweichungen führen.

Die Elektroden werden vorzugsweise stets jeweils mit der Arzneimittelzubereitung/Probe derart verbunden, dass ein direkter Kontakt der Elektroden zueinander verhindert wird und die Elektroden mit der Arzneimittelzubereitung/Probe eine Reihenschaltung bilden. Ein in eine Elektrode eingeprägter Strom fließt folglich durch die Arzneimittelzubereitung/Probe in die andere(n) Elektrode(n). Es können mehr als zwei Elektroden vorgesehen sein. Hierdurch können unterschiedliche Widerstände derselben Arzneimittelzubereitung/Probe in unterschiedliche Richtungen durch die Arzneimittelzubereitung/Probe bestimmt werden. Hierdurch können in vorteilhafter Weise inhomogene Wasserverteilungen identifiziert oder berücksichtigt werden. Bevorzugt und im Folgenden beschrieben sind jedoch stets Ausführungen mit zwei Elektroden.

Vorzugsweise werden oder sind in der Messkammer zu einer bestimmten Formulierung und/oder Darreichungsform, insbesondere Silhouette oder Form der Oberfläche, der Arzneimittelzubereitung/Probe korrespondierende Elektroden montiert. Vorzugweise weisen die Elektroden Kontaktflächen zur Verbindung mit der Arzneimittelzubereitung/Probe auf, die jeweils zumindest im Wesentlichen als Negativbild eines Oberflächenabschnitts der Arzneimittelzubereitung/Probe ausgebildete sind. Hierdurch wird für die jeweils vorliegende Darreichungsform der Arzneimittelzubereitung/Probe sichergestellt, dass eine definierte und ausreichend große Kontaktfläche zwischen der Arzneimittelzubereitung/Probe und den Elektroden erzeugt werden kann, was der Reproduzierbarkeit und Exaktheit der Bestimmung des Wassergehalts der Arzneimittelzubereitung/Probe zuträglich ist.

Gemäß einer bevorzugten Ausführungsform ist vorgesehen, dass die Elektroden ausgetäuscht werden, wenn die Darreichungsform der Arzneimittelzubereitung/Probe für eine weitere Messung geändert wird. Die Elektroden sind also bevorzugt austauschbar, insbesondere automatisch durch ein Wechselsystem, ein Revolversystem o. dgl.

Die Elektroden werden oder sind vorzugsweise mit einer Messeinrichtung zur Messung des Widerstands der Arzneimittelzubereitung/Probe verbunden. Bei der Messeinrichtung kann es sich insbesondere um ein Elektrometer handeln. Das Messprinzip eines Elektrometers hat sich als besonders vorteilhaft für eine exakte und reproduzierbare sowie schnelle Ermittlung des Wassergehalts von Arzneimittelzubereitungen/Proben gezeigt. Hierbei ist vorgesehen, dass an der Arzneimittelzubereitung/Probe eine definierte Spannungsänderung angelegt wird und ein Differenzstrom durch die Arzneimittelzubereitung/Probe gemessen wird, der sich durch die Spannung oder Spannungsänderung ergibt. Grundsätzlich sind jedoch auch andere Verfahren anwendbar, beispielsweise solche, bei denen ein (Differenz-)Strom eingeprägt wird und die sich hierdurch ergebene (Differenz-)Spannung gemessen wird.

Zur Bestimmung des Widerstands der Arzneimittelzubereitung/Probe wird vorzugsweise ein durch die Arzneimittelzubereitung/Probe fließender Strom und eine über die Arzneimittelzubereitung/Probe abfallende Spannung ermittelt. Durch Division der jeweiligen Spannung durch den jeweiligen Strom kann der jeweilige Widerstand berechnet werden.

Vorzugsweise werden einer solchen oder entsprechenden Berechnung keine absoluten Strom- und Spannungswerte zugrunde gelegt. Stattdessen ist es besonders bevorzugt, dass die Bestimmung des Widerstands auf Differenzspannungen und Differenzströmen basiert. Hierbei kann die Spannung geändert werden, die an den Elektroden bzw. über der Arzneimittelzubereitung/Probe anliegt. Diese Spannungsänderung entspricht einer Differenzspannung oder Spannungsdifferenz. Aufgrund der Spannungsänderung erfolgt, insbesondere gemäß dem ohmschen Gesetz, eine Änderung des durch die Arzneimittelzubereitung/Probe fließenden Stroms, also ein Differenzstrom bzw. eine Stromdifferenz. Der Quotient aus der Differenzspannung zum Differenzstrom, also die Differenzspannung geteilt durch den korrespondierenden Differenzstrom, entspricht ebenfalls dem Widerstand der Arzneimittelzubereitung/Probe.

Vorteil dieses Verfahrens entgegen einer klassischen Bestimmung des Widerstands mit absoluten Strom- und Spannungsgrößen liegt darin, dass das vorliegende Bestimmungsprinzip unabhängig von Störgrößen ist, die zu einem konstanten Fehler oder Offset führen. Beispielsweise wird vermieden, dass eine in die Messeinrichtung eingekoppelte Gleichspannung oder ein durch Kontaktübergänge o. dgl. erzeugter Gleichanteil die Messung beeinflusst oder verfälscht. Dies hat sich als besonders vorteilhaft für eine exakte Bestimmung des Widerstands und im weiteren des Wassergehalts erwiesen.

Zur Messung wird bevorzugt die an oder über der Arzneimittelzubereitung/Probe anliegende Spannung geändert und die sich aus dieser Spannungsänderung ergebene Änderung des durch die Arzneimittelzubereitung/Probe fließenden Stroms ermittelt, insbesondere gemessen. Alternativ oder zusätzlich kann der durch die Arzneimittelzubereitung/Probe fließende Strom geändert werden und eine sich hierdurch einstellende Spannungsänderung wird ermittelt bzw. gemessen. Grundsätzlich ist es jedoch auch möglich, den durch die Arzneimittelzubereitung/Probe fließenden Strom und die an der Arzneimittelzubereitung/Probe anliegende Spannung gleichzeitig oder im Wechsel zu ändern, wobei vorzugsweise die Beziehungen zwischen dem Strom und der Spannung bzw. der Stromänderung und der Spannungsänderung stets durch den Widerstand der Arzneimittelzubereitung/Probe bestimmt oder beeinflusst ist/sind und folglich durch die jeweiligen Strom-Spannungsbeziehungen der Widerstand ermittelt, insbesondere berechnet, werden kann.

Besonders bevorzugt ist eine Ermittlung des Widerstands, indem die Polarität, die Richtung bzw. das Vorzeichen einer hierzu angelegten Spannung oder eines hierzu eingeprägten Stroms mehrfach umgekehrt wird. Dies erfolgt jedoch vorzugsweise nur zur Detektion und Elemination von Störgrößen und nicht zur Beeinflussung der Ergebnisse durch ein kapazitives oder induktives Verhalten. Vor diesem Hintergrund wird vorzugsweise ein Polarisationswechsel lediglich im Abstand von einer oder mehreren Sekunden durchgeführt und/oder in der oder für die Messung eine Einschwingzeit von einer oder mehreren Sekunden abgewartet.

Gemäß der Erfindung wird die Arzneimittelzubereitung/Probe innerhalb der Messkammer mit einer vorgegebenen Andruckkraft der Elektroden auf die Arzneimittelzubereitung/Probe in unmittelbaren Kontakt mit der Arzneimittelzubereitung/Probe gebracht. Hierzu kann ein Spannmittel vorgesehen sein, das die Elektroden mit einer vorgegebenen Kraft an die Arzneimittelzubereitung/Probe andrücken kann. Alternativ oder zusätzlich kann ein Drucksensor vorgesehen sein, mit dem der Anpressdruck der Kontakte an die Arzneimittelzubereitung/Probe kontrolliert, gesteuert und/oder geregelt werden kann. Hierdurch wird in vorteilhafterweise vermieden, dass die Eigenschaften der Arzneimittelzubereitung/Probe und der elektrischen Kontakte der Elektroden mit der Arzneimittelzubereitung/Probe, insbesondere nach einem Probenwechsel bzw. Austausch der Arzneimittelzubereitung/Probe, variieren und ggfs. zu Unsicherheiten und Toleranzen führen.

Der Wassergehalt oder die relative Feuchtigkeit einer die Arzneimittelzubereitung/Probe umgebenden Atmosphäre innerhalb der Messkammer wird erfindungsgemäß eingestellt oder vorgegeben.

Hierdurch wird vermieden, dass eine Wasseraufnahme in die Arzneimittelzubereitung/Probe oder eine Wasserabgabe aus der Arzneimittelzubereitung/Probe vor oder während der Messung zu Ungenauigkeiten führt. Dies gilt insbesondere für Kalibriermessungen.

Insbesondere ist vorgesehen, dass in der Messkammer ein Konditionierungsmittel vorgesehen oder angeordnet wird, insbesondere ein Trocknungsmittel, Befeuchtungsmittel und/oder eine gesättigte Salzlösung o. dgl.

Vorzugsweise wird eine relative Feuchtigkeit der Atmosphäre innerhalb der Messammer, insbesondere eine (relative) Luftfeuchtigkeit in der Messkammer vorgegeben und/oder eingestellt, insbesondere mit dem Konditionierungsmittel. Dies kann in einer bevorzugten Ausführungsform so erfolgen, dass eine Aufnahme oder Abgabe von Wasser durch die Arzneimittelzubereitung/Probe reduziert oder vermieden wird. Hierdurch kann eine Beeinflussung des Widerstands durch die Messumgebung in vorteilhafter Weise vermieden werden.

Ferner ist bevorzugt, dass in der Kammer dieselbe Zusammensetzung der Atmosphäre verwendet wird, wie diejenige, die bei einem beabsichtigten Verpacken der Arzneimittelzubereitung/Probe verwendet wird. Hierdurch kann eine verbesserte Genauigkeit der Bestimmung des Wassergehalts dadurch erreicht werden, dass ausgeschlossen wird, dass die Arzneimittelzubereitung/Probe umgebende Gasphase oder Atmosphäre den Messvorgang beeinflusst, beispielsweise durch physikalische und/oder chemische Veränderung der Arzneimittelzubereitung/Probe. Die Messkammer kann hierzu mit einem Gas, insbesondere einem Schutzgas, als Atmosphäre gefüllt werden oder füllbar sein.

Gemäß einem Aspekt ist besonders bevorzugt, dass für die Bestimmung des Wassergehalts der Arzneimittelzubereitung/Probe nach Abschluss einer Offenlagerungsstudie, bei der die Arzneimittelzubereitung/Probe definierten Umgebungsbedingungen über ein bestimmte Zeitspanne ausgesetzt worden ist, diese Umgebungsbedingungen innerhalb der Kammer beibehalten, eingestellt oder vorgegeben werden, um den Einfluss der Umgebungsbedingungen der Offenlagerungsstudie während der Messung nicht zu verfälschen. Beispielsweise wird in einer Offenlagerungsstudie eine ganz bestimmte Temperatur und eine ganz bestimmte (relative) Luftfeuchtigkeit der Umgebung eingestellt und die Arzneimittelzubereitung/Probe über eine vorgegebene Zeitspanne diesen Umgebungsbedingungen offen ausgesetzt. Für die Messung ist dann bevorzugt, dass in der Messkammer dieselben Umgebungsbedingungen, insbesondere also dieselbe Temperatur und/oder (relative) Luftfeuchtigkeit eingestellt wird/werden. Besonders vorteilhaft ist die Verwendung dieses Aspekts bei, zu oder im Zusammenhang mit Kalibriermessungen bzw. Kalibrierschritten oder allgemein der Kalibrierung. Hierbei kann ein Zusammenhang zwischen dem Widerstand und dem Wassergehalt ermittelt werden.

Mit den Ergebnissen, also mit dem Wassergehalt oder einem hierzu korrespondierenden Wert, der mit dem vorschlagsgemäßen Verfahren bestimmt worden ist, kann im Folgenden die Haltbarkeit der Arzneimittelzubereitung/Probe, insbesondere bei Lagerung in einer bestimmten Verpackungsform, berechnet und/oder prognostiziert werden. Hierbei ist es bevorzugt, das zusätzlich zum Wassergehalt der Arzneimittelzubereitung/Probe der Wassergehalt des Verpackungsmaterials und der bei dem Verpackungsprozess eingeschlossenen Atmosphäre bestimmt und daraufhin mit einem Sorptionsmodell o. dgl. die Feuchtigkeitsaufnahme durch die Verpackung hindurch und der Feuchtigkeitsaustausch zwischen den unterschiedlichen Materialien, insbesondere also der Verpackung, der Atmosphäre und der Arzneimittelzubereitung/Probe, innerhalb der Verpackung simuliert wird. Hierdurch können Rückschlüsse auf die Wasseraufnahme durch die Arzneimittelzubereitung/Probe und/oder die Haltbarkeit bzw. Degeneration der Arzneimittelzubereitung/Probe gezogen werden.

In einem weiteren Aspekt wird der Widerstand einer Arzneimittelzubereitung/Probe zur Bestimmung des Wassergehalts der Arzneimittelzubereitung/Probe und/oder zur Prognose der Haltbarkeit der Arzneimittelzubereitung/Probe, vorzugsweise in einer bestimmten Verpackungsform, bestimmt oder berechnet.

Ein weiterer, auch unabhängig realisierbarer Aspekt der vorliegenden Erfindung betrifft eine Messkammer zur Ermittlung eines Widerstands zum Bestimmen eines Wassergehalts einer Arzneimittelzubereitung und/oder eines Verpackungsmaterials gemäß Anspruch 7. Die Messkammer weist mindestens zwei Elektroden auf und ist dazu ausgebildet, die Arzneimittelzubereitung/Probe aufzunehmen und derart mit den Elektroden in unmittelbaren Kontakt zu bringen, dass die Elektroden durch die Arzneimittelzubereitung/Probe miteinander elektrisch verbunden sind. Ferner ist vorgesehen, dass die Messkammer luftdicht verschließbar ist, so dass die Messkammer eine die Arzneimittelzubereitung/Probe umgebende Atmosphäre einschließt, wobei die Messkammer dazu eingerichtet ist, einen Wassergehalt der von der Messkammer eingeschlossenen Atmosphäre einzustellen.

Zur Bestimmung des Wassergehalts einer nicht-kompakten Arzneimittelzubereitung/Probe, also eines Pulvers, Granulats o. dgl., kann mindestens eine der Elektroden eine Aufnahme oder Kavität aufweisen, die die Arzneimittelzubereitung/Probe aufnehmen kann, wobei die Aufnahme dazu ausgebildet ist, einen Kontakt der Arzneimittelzubereitung/Probe zu den Elektroden zu ermöglichen, ohne dass die Elektroden unmittelbar elektrisch miteinander verbunden werden. Insbesondere bildet eine der Elektroden eine Bodenplatte der Aufnahme und die zweite Elektrode kann als Stempel in die Aufnahme eintreten und die Arzneimittelzubereitung/Probe in der Aufnahme komprimieren. Hier sind jedoch auch andere Lösungen möglich.

Vorzugsweise weist die Messkammer ein Konditionierungsmittel zur Beeinflussung oder Einstellung der (relativen) Feuchte der Atmosphäre auf. Vorzugsweise sind innerhalb der Messkammer neben den Elektroden geeignete Mittel, insbesondere ein Behältnis, ein Befestigungsmittel o. dgl. vorgesehen, wodurch das Konditionierungsmittel innerhalb der Kammer derart positionierbar ist, dass der Wassergehalt, die Feuchte bzw. relative Feuchtigkeit der Atmosphäre durch das Konditionierungsmittel beeinflussbar oder einstellbar ist, das Konditionierungsmittel jedoch keinen unmittelbaren Kontakt zu den Elektroden bzw. zu der Arzneimittelzubereitung/Probe erhält, um eine Verfälschung der Messung auszuschließen.

Die Elektroden sind vorzugsweise, zumindest im Bereich der Kontaktflächen für die Arzneimittelzubereitung/Probe, innerhalb der Messkammer angeordnet oder anordenbar. Die Kammer kann durch eine, insbesondere luftdichte, Kammerwand begrenzt sein. Die Kammer ist vorzugsweise öffenbar, insbesondere teilbar, so dass die Arzneimittelzubereitung/Probe in die Kammer eingebracht und mit den Elektroden verbunden werden kann.

Die Elektroden, die in der Messkammer vorgesehen sind, sind vorzugsweise zu einer Form und/oder Beschaffenheit der Arzneimittelzubereitung/Probe korrespondierend ausgebildet. Insbesondere ist vorgesehen, dass die Elektroden Kontaktflächen aufweisen, die komplementär zur äußeren Form oder zu Abschnitten der äußeren Form der jeweiligen Arzneimittelzubereitung/Probe gebildet sind. Hierdurch wird erreicht, dass eine reproduzierbare und ausreichend große Kontaktfläche zwischen den Elektroden und der Arzneimittelzubereitung/Probe erreicht werden kann.

Die Elektroden sind in der Messkammer vorzugsweise austauschbar gehalten. Hierdurch wird in vorteilhafter Weise erreicht, dass zu der Form und/oder Beschaffenheit der Arzneimittelzubereitung/Probe korrespondierende Elektroden wählbar und/oder in der Messkammer montierbar sind.

Ferner ist bevorzugt, dass die Elektroden ein Spannmittel und/oder eine Kraftmesseinrichtung aufweisen, wodurch die Elektroden mit einer vorgebaren und/oder reproduzierbaren Kraft an die Arzneimittelzubereitung/Probe andrückbar sind. Dies ist sowohl der Reproduzierbarkeit der Bestimmung des Wassergehalts der Arzneimittelzubereitung/Probe zuträglich als auch vorteilhaft dadurch, dass eine mechanische Beeinträchtigung der Arzneimittelzubereitung/Probe durch zu große auf diese wirkende Kräfte vermieden werden kann.

Die Elektroden sind vorzugsweise relativ zueinander bewegbar in der Messkammer gehalten, so dass die Elektroden an die zwischen den Elektroden angeordnete Arzneimittelzubereitung/Probe angedrückt werden können. Insbesondere ist also mindestens eine der Elektroden verfahrbar, verschiebbar oder auf sonstige Weise bewegbar. Hierdurch können die Elektroden zum Einlegen oder Einsetzen der Arzneimittelzubereitung/Probe auseinander bewegt werden und nach Platzierung der Arzneimittelzubereitung/Probe zwischen den Elektroden jeweils in unmittelbaren Kontakt mit der Arzneimittelzubereitung/Probe gebracht werden. Hierzu werden die Elektroden vorzugsweise an die Arzneimittelzubereitung/Probe mit einer Andrucckraft angedrückt.

Die Messkammer weist vorzugsweise eine Messeinrichtung auf, die zur Messung des Widerstands der Arzneimittelzubereitung/Probe ausgebildet ist. Die Messeinrichtung kann ein Elektrometer sein oder aufweisen und/oder zur Bestimmung des Widerstands auf Basis einer Messung von Differenzspannungen und Differenzströmen ausgebildet sein.

Die Elektroden sind vorzugsweise elektrisch mit der Messeinrichtung verbunden. Hierzu können die Elektroden mit elektrisch leitenden Kabeln verbunden sein, die insbesondere aus der Messkammer herausführen und die Elektroden elektrisch mit der Messeinrichtung verbinden. Die Kabel sind vorzugsweise geschirmt und/oder antistatisch, um Verfälschungen der jeweiligen Messung zu verhindern. Die Messeinrichtung ist vorzugsweise außerhalb der Kammer bzw. Atmosphäre angeordnet.

Die Messkammer weist vorzugsweise einen Feuchtesensor zur Bestimmung der (relativen) Feuchtigkeit der Atmosphäre auf. Hierdurch kann kontrolliert werden, ob die (relative) Feuchtigkeit der Atmosphäre durch das Konditionierungsmittel oder auf sonstige Weise korrekt eingestellt ist. Alternativ oder zusätzlich kann durch den Feuchtesensor eine Steuerung oder Regelung der (relativen) Feuchtigkeit der Atmosphäre erfolgen. Beispielsweise kann in Abhängigkeit von einem Messwert des Feuchtesensors die Menge oder Wirkung des Konditionierungsmittels beeinflusst werden, insbesondere durch Änderung der Menge von Konditionierungsmittel, durch (teilweises) Abdecken und/oder durch Austausch bzw. Bewegung der Atmosphäre.

Es ist bevorzugt, dass die Feuchtebestimmungseinrichtung bzw. das Zuordnungsmittel unerwünschte, sogenannte parasitäre, Effekte der Messanordnung einschließlich der Zuleitungen und der Elektroden berücksichtigt oder eliminiert. Hierzu kann eine Kalibrierung erfolgen. Vorzugsweise wird zur Kalibrierung ein Kurzschluss zwischen den Elektroden hergestellt, wodurch Widerstandsanteile der Messanordnung bestimmt oder gemessen werden, die im Folgenden bei der Bestimmung des Widerstands der Arzneimittelzubereitung/Probe berücksichtigt werden, insbesondere in Abzug gebracht werden.

Es ist weiter bevorzugt, dass die Messkammer eine Feuchtebestimmungseinrichtung zur Bestimmung und, vorzugsweise, Ausgabe des Wassergehalts der Arzneimittelzubereitung/Probe aufweist, der zu dem bestimmten Widerstand korrespondiert. Die Feuchtebestimmungseinrichtung kann zu diesem Zweck das Zuordnungsmittel aufweisen oder auf das Zuordnungsmittel zugreifen und nach Messung des Widerstands ein zu den bestimmten Widerstand korrespondierenden Wassergehalt ermitteln, beispielsweise durch Auslesen eines entsprechenden Werts aus einer Zuordnungstabelle oder durch Einsetzen in eine Zuordnungsfunktion.

Ferner weist die Messkammer vorzugsweise eine Haltbarkeitsbestimmungseinrichtung auf, die dazu ausgebildet ist, mit dem Wassergehalt eine Haltbarkeit der Arzneimittelzubereitung/Probe in einer bestimmten Verpackung zu berechnen und, vorzugsweise, auszugeben. Es ist also insbesondere vorgesehen, das ein oder mehrere Messungen der Messeinrichtung mit ein oder mehreren Proben der Arzneimittelzubereitung/Probe automatisch ausgewertet und in Kombination mit Eigenschaften von Verpackungen dazu verwendet werden, die Haltbarkeit der in einer oder mehreren bestimmten Verpackungen verpackten Arzneimittelzubereitung/Probe zu prognostizieren und, vorzugsweise, durch Vergleich der Prognosen eine vorteilhafte, geeignete Verpackung auszuwählen oder eine entsprechende Auswahl geeigneter Verpackungen zur Verfügung zu stellen.

Ein Wassergehalt im Sinne der vorliegenden Erfindung ist vorzugsweise der Wasseranteil, der einer Substanz bzw. der Arzneimittelzubereitung/Probe durch Trocknung entzogen werden kann. Insbesondere wird der Begriff "Wassergehalt" so verstanden, dass er dem Wassergehalt entspricht, der mit den bekannten Referenzmethoden wie der Dynamic Vapor Sorption-Wägung (DVS-Wägung), der Karl-Fischer-Titration oder der Gravimetrischen Trockenschrank Methode bestimmt wird.

Es ist möglich, dass bei der Bestimmung des elektrischen Widerstands sowohl freies als auch gebundenes Wasser berücksichtigt wird, da beide zur Leitfähigkeit beitragen: Freies Wasser z.B. durch direkten lonentransport und Freisetzung weiterer Ionen; Gebundenes durch thermisch aktivierten direkten lonentransport sowie durch Freisetzung von Ionen, z.B. über eine Veränderung der Kristallstruktur oder eine Veränderung des Hintergrund-Kristallfeldes und damit der Fermi-Energie.

Elektroden im Sinne der vorliegenden Erfindung sind vorzugsweise Elemente mit elektrisch leitenden Oberflächen, die dazu ausgebildet sind, einen unmittelbaren bzw. galvanischen Kontakt mit einer Substanz, insbesondere der Arzneimittelzubereitung/Probe, herzustellen. Die Elektroden können aus Metall bestehen oder Metall aufweisen, vorzugsweise Edelmetall wie Gold oder Platin. Grundsätzlich kommen jedoch auch andere elektrisch leitfähige Materialien in Frage.

Vorzugweise ist mit dem Begriff "Widerstand" im Sinne der vorliegenden Erfindung stets der. insbesondere elektrische und/oder ohmsche, Widerstand der Arzneimittelzubereitung/Probe, insbesondere zwischen den Elektroden, gemeint, außer es ergibt sich etwas anderes aus dem Zusammenhang oder der Erläuterung. Ein Widerstand im Sinne der vorliegenden Erfindung ist vorzugsweise ein sogenannter Gleichstrom-Widerstand bzw. Realteil einer Impedanz. Alternativ oder zusätzlich kann jedoch auch ein reaktives Verhalten berücksichtigt werden, beispielsweise ein kapazitives Verhalten der Arzneimittelzubereitung/Probe.

Eine Arzneimittelzubereitung/Probe im Sinne der vorliegenden Erfindung ist vorzugsweise eine einen oder mehrere Wirkstoffe und/oder einen oder mehrere Hilfsstoffe aufweisende Substanz.

Wirkstoffe sind vorzugsweise physiologisch aktive Substanzen, die dazu geeignet sind, auf einen Körper eines Lebewesens oder dessen Funktionen förderlich, therapierend, heilend oder auf sonstige Weise einzuwirken.

Hilfsstoffe sind insbesondere auch Arzneiträgerstoffe oder Wirkstoffträgerstoffe. Hilfsstoffe sind vorzugsweise pharmakologisch und/oder toxikologisch inert. Hilfsstoffe können dazu ausgebildet sein, den Wirkstoff zu tragen, die Wirkung des Wirkstoffs zu beeinflussen oder zu verstärken, der Arzneimittelzubereitung/Probe seine Form zu geben, Fertigungsschritte zu verbessern, die Dauer und Geschwindigkeit Wirkstofffreigabe zu steuern und/oder die Arzneimittelzubereitung/Probe mechanisch bzw. chemisch zu stabilisieren, um eine ausreichende Haltbarkeit zu erreichen.

Die Arzneimittelzubereitung/Probe im Sinne der vorliegenden Erfindung ist vorzugsweise ein Feststoff, insbesondere bei Raumtemperatur oder 300 K. Dieser Feststoff liegt vorzugsweise in kompakter Form vor, insbesondere Kapselform oder Tablettenform, kann jedoch auch granular, als Granulat, Pulver, in Form von Kügelchen oder in sonstigen Formen vorliegen. Die Arzneimittelzubereitung/Probe kann insbesondere auch eine Salbe, ein Gel, eine Creme, eine Paste, aber auch eine Flüssigkeit oder ein Gas sein, obwohl eine feste Arzneimittelzubereitung/Probe bevorzugt ist.

Der Begriff "Atmosphäre" im Sinne der vorliegende Erfindung ist vorzugsweise so zu verstehen, dass es sich hierbei um eine gasförmige, insbesondere bei Normaldruck oder Umgebungsdruck gasförmige, Substanz handelt, die innerhalb der Messkammer die Arzneimittelzubereitung/Probe und/oder die Elektrode(n) umgibt. Insbesondere handelt es sich hierbei um Luft, Stickstoff und/oder Schutzgas. Die Atmosphäre kann gasförmiges Wasser aufweisen, wodurch sich je nach Sättigung der Atmosphäre mit Wasser eine (relative) Feuchtigkeit der Atmosphäre ergibt.

Vorzugsweise ist bei der vorliegenden Erfindung mit dem Begriff "Strom", vorzugsweise auch bei zusammengesetzten Begriffen wie "Differenzstrom" oder "Stromdifferenz", stets der Strom gemeint, der durch die Arzneimittelzubereitung/Probe fließt, außer gegenteiliges ist explizit erwähnt oder ergibt sich aus dem Zusammenhang.

Bei Verwendung des Begriffs "Spannung", auch bei zusammengesetzten Begriffen wie "Differenzspannung" oder "Spannungsdifferenz", ist bei der vorliegenden Erfindung vorzugsweise stets eine über der Arzneimittelzubereitung/Probe abfallende oder anliegende Spannung gemeint, falls sich nichts gegenteiliges aus dem Zusammenhang ergibt oder erläutert ist.

Ein weiterer Aspekt, der jedoch nicht Gegenstand der Erfindung ist, betrifft eine Verpackung, vorzugsweise für granulare Trockenmittel, wobei die Verpackung vorzugsweise zumindest abschnittsweise wasserdampfdurchlässig ist und eine feuchtigkeitsempfindliche Widerstandseinrichtung aufweist, bei der ein elektrischer Widerstand mit Veränderung einer Feuchtigkeit oder einem Wassergehalt der Widerstandseinrichtung variiert, und wobei die Widerstandseinrichtung an unterschiedlichen Abschnitten, bevorzugt außerhalb der Verpackung elektrisch kontaktierbar ist, so dass die Kontakte bzw. Abschnitte durch die Widerstandseinrichtung untereinander derart elektrisch verbunden sind, dass der elektrische Widerstand der Widerstandseinrichtung messbar ist. Die Verpackung weist vorzugsweise Trockenmittel auf.

Es hat sich herausgestellt, dass entsprechende Verpackungen dazu genutzt werden, können, durch Messung des Widerstands auf einfache, schnelle und kostengünstige Weise auf den Zustand des mit der Verpackung verpackten oder zu verpackenden Trocknungsmittels oder einer sonstigen Substanz rückschließen zu können.

Vorzugsweise ist die Widerstandseinrichtung folienartig und/oder streifenartig. Weiter ist bevorzugt, dass die Verpackung ganz oder teilweise durch die Widerstandseinrichtung gebildet ist.

Es ist bevorzugt, dass die Widerstandseinrichtung durch einen Beilaufstreifen gebildet ist, vorzugsweise wobei der Beilaufstreifen entlang der Verpackung in diese eingebracht und/oder an dieser angebracht ist.

Die Widerstandseinrichtung weist vorzugsweise orientiertes Polyamid und/oder ein sonstiges Material auf oder ist hieraus gebildet, das eine starke Feuchteabhängigkeit seines elektrischen Widerstands aufweist, vorzugsweise so dass sich der elektrische Widerstand zwischen einem trockenen und einem (luft)feuchten Zustand der Widerstandseinrichtung, gemessen gemäß IEC 60093, um mehr als Faktor 10 oder 100, insbesondere mehr als Faktor 500 unterscheidet.

Vorzugsweise ist die Widerstandseinrichtung so angeordnet bzw. die Verpackung so ausgebildet, dass der mit der Verpackung zu verpackende Inhalt in Verbindung oder Kontakt mit der Widerstandseinrichtung steht bzw. Wasser oder Wasserdampf mit der Widerstandseinrichtung austauschen kann.

Ein weiterer Aspekt, der jedoch nicht Gegenstand der Erfindung ist, betrifft ein Verfahren zur Herstellung einer bevorzugt zumindest abschnittsweise wasserdampfdurchlässigen Verpackung, wobei eine Widerstandseinrichtung, die einen feuchtigkeitsabhängigen elektrischen Widerstand aufweist, an und/oder in der Verpackung vorgesehen wird.

Weiter sind insbesondere folgende Aspekte vorteilhaft:
Der Feuchtegehalt von Packmitteln, Verpackungen und Devices wird mit Hilfe der elektrischen Leitfähigkeit ermittelt.

Die Messung bzw. Ermittlung des Feuchtegehalts von pharmazeutischen Produkten, insbesondere von festen Formen, in besonderem Maße von Tablettenkernen, Filmtabletten, Granulaten, Pulvern und Kapseln erfolgt mit Hilfe der elektrischen Leitfähigkeit.

Die Genauigkeit der Messung bzw. Ermittlung wird vorzugsweise durch Sicherstellung der Konditionierung der Probe in einer abgedichteten Kammer verbessert.

Die Genauigkeit der Messung bzw. Ermittlung bzw. Kalibrierung wird vorzugsweise durch Bestückung der Kammer mit einem vorkonditionierten Trockenmittel erreicht.

Die Genauigkeit der Messung bzw. Ermittlung bzw. Kalibrierung kann durch Nutzung einer Kombination von gesättigter Salzlösung und Trockenmittel, z.B. Silikagel, verbessert werden.

Der Genauigkeit der Messung bzw. Ermittlung bzw. Kalibrierung kann durch geeignete Elektroden bzw. Kontaktmaterialien verbessert werden, insbesondere wobei: Elektroden bzw. Kontaktmaterialien austauschbar, in der Form der Probe anpassungsfähig bzw. angepasst und gleichzeitig leitfähig sind.

Die Genauigkeit der Messung bzw. Ermittlung bzw. Kalibrierung kann durch Verwendung von Druckstempeln (insbesondere form-identisch zu Tablettierwerkzeug) als Elektrode verbessert werden.

Die Messgenauigkeit kann durch Kombination zwischen DVS und beispielsweise CRDS bzw. Spurenfeuchte verbessert werden.

Es erfolgt vorzugsweise eine Messung des Feuchtegehalts von Coatings bzw. die Messung wird hierauf erweitert.

Es erfolgt vorzugsweise eine Feuchtegehaltsmessung durch Oberflächenmessungen, beispielsweise an Filmtabletten und Coatings.

Es werden vorzugsweise vereinfachte "Plattform-Lösungen" für Produkte mit dem gleichen Coating bereitgestellt.

Die Genauigkeit der Messung bzw. Ermittlung bzw. Kalibrierung wird vorzugsweise durch Einsatz von Coatings mit besonders hoher Feuchteabhängigkeit des Widerstandes als "aufgetragene Sensoren" verbessert. Das Coating liegt an der Arzneimittelzubereitung an, wodurch eine Widerstandsmessung des Coatings verwendet werden kann, auf den Wassergehalt der Arzneimittelzubereitung rückzuschließen.

Das vorschlagsgemäße Verfahren bzw. die Widerstandsmessung wird vorzugsweise verwendet, um eine Probenstabilität zu ermitteln, insbesondere wobei eine Korrelation zwischen Widerstand und Sprödigkeit, insbesondere einer Gelatinekapsel o.dgl., ermittelt wird.

Das vorschlagsgemäße Verfahren bzw. die Widerstandsmessung wird vorzugsweise zur Bestimmung einer Korrelation zwischen Widerstand und Härte einer Tablette bzw. Kapsel oder sonstigen Arzneimittelzubereitung verwendet.

Das vorschlagsgemäße Verfahren bzw. die Widerstandsmessung wird vorzugsweise mit Hilfe eines Beilaufstreifens, z.B. aus OPA, an losen Trockenmitteln angewendet.

Das vorschlagsgemäße Verfahren bzw. die Widerstandsmessung wird vorzugsweise auf lose Formulierungen, z.B. Pulver, Granulate, sowie komprimierte Formulierungen, z.B. Kompaktate, Pellets, Tabletten, Mini-Tabletten z.B. in Stick Packs sowie auf Wirk- und Hilfsstoffe angewenet.

Durch Verdichten der losen Proben durch Pressen wird die Genauigkeit verbessert.

Probenstempel werden vorzugsweise als elektrische Kontakte verwendet.

Eine Druckabhängigkeit des elektrischen Widerstands als Fehlerquelle wird vorzugsweise durch Arbeiten bei einem konstanten Druck auf die Probe insbesondere in einem sog. "Druckplateau" zumindest im Wesentlichen eliminiert.

Das vorschlagsgemäße Verfahren wird vorzugsweise zu Feuchtegehaltsmessungen an Hilfsstoffen, Wirk- und Rohstoffen verwendet.

Das vorschlagsgemäße Verfahren wird vorzugsweise zur Kontrolle von Härte, Sprödigkeit und/oder Klebrigkeit an Kapseln verwendet.

Die Messung des Feuchtegehalts von Packmitteln erfolgt vorzugsweise indirekt, z.B. bei Verpackungsfolien, bevorzugt mit Hilfe einer "Abklatsch-Messung" an einem aufliegenden Material, beispielsweise oPA.

Das vorschlagsgemäße Verfahren wird vorzugsweise zur Messung unrelaxierter Proben (z.B. bei Maschinenstillständen) verwendet.

Die Genauigkeit der Messung bzw. Ermittlung bzw. Kalibrierung wird vorzugsweise durch Konditionierung der Tablette in der Messkammer und veränderbares Luftvolumen für Kalibrierung und Messung verbessert.

Die Genauigkeit der Messung bzw. Ermittlung bzw. Kalibrierung wird vorzugsweise durch Überprüfung der r.F. mit Stechfühler verbessert.

Die Genauigkeit der Messung bzw. Ermittlung bzw. Kalibrierung wird vorzugsweise durch Minimierung der Kriechströme durch spezielles Handling (z.B. Reinigung, Handschuhe, kurze Kabel) verbessert.

Eine Zeitersparnis bei der Kalibrierungsmessung wird vorzugsweise durch Anwendung einer Multisample-DVS-Apparatur zur parallelen Konditionierung von Proben erreicht.

Zur Verbesserung der Genauigkeit der Messung bzw. Ermittlung bzw. Kalibrierung wird vorzugsweise durch Komprimierung der Proben in der Messkammer die Kontaktfläche zwischen Probe und Elektroden vergrößert.

Vorzugsweise wird die Genauigkeit durch Messen im Druck-Plateaubereich (optimale Verdichtung des Pulvers) mit einer Pulvermesskammer erreicht.

Weitere Vorteile, Aspekte, Merkmale und Eigenschaften der vorliegenden Erfindung ergeben sich aus den Ansprüchen und aus der folgenden Beschreibung bevorzugter Ausführungsformen anhand der Zeichnung. Es zeigt:
- Fig. 1: eine vorschlagsgemäße Messkammer;
- Fig. 2: eine vorschlagsgemäße Messeinrichtung;
- Fig. 3: eine vorschlagsgemäße Feuchtebestimmungseinrichtung;
- Fig. 4: eine alternative Feuchtebestimmungseinrichtung;
- Fig. 5A bis 5E: vorschlagsgemäße Elektroden;
- Fig. 6: eine vorschlagsgemäße Elektrodeneinrichtung zur Messung des Widerstands einer pulverförmigen oder granulären Arzneimittelzubereitung/Probe;
- Fig. 7: ein vorschlagsgemäßes Verpackungssystem; und
- Fig. 8: einen Schnitt des vorschlagsgemäßen Verpackungssystems gemäß Schnittlinie VIII-VIII aus Fig. 7.

In den Figuren werden für gleiche oder ähnliche Teile dieselben Bezugszeichen verwendet, wobei gleiche oder ähnliche Eigenschaften und Vorteile erreicht werden können, auch wenn von einer wiederholten Beschreibung abgesehen wird.

Im Folgenden wird die Erfindung anhand konkreter Ausführungsbeispiele betreffend eine oder mehrere Arzneimittelzubereitungen im Detail erläutert. Vorzugsweise ist die vorliegende Erfindung jedoch auch in diesem Zusammenhang nicht auf "Arzneimittelzubereitungen" beschränkt. Daher kann der Begriff "Arzneimittelzubereitungen" vorzugsweise durch den Begriff "Probe", den Begriff "Verpackungsmaterial" oder die Kombination "Arzneimittelzubereitung und/oder Verpackungsmaterial" ersetzt werden. Dies gilt jedenfalls soweit der jeweilige Zusammenhang einer Ersetzung nicht denklogisch entgegensteht. Verwendet wird daher im Folgenden der Term "Arzneimittelzubereitung/Probe". Ergänzend wird auf die Definitionen und Erläuterungen aus dem allgemeinen Beschreibungsteil verwiesen.

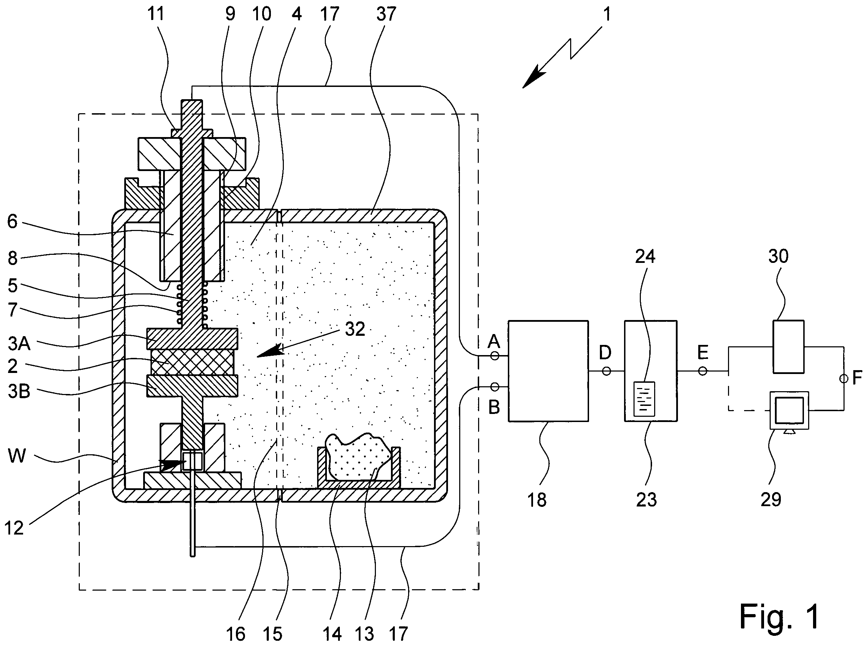

Fig. 1 zeigt eine Messkammer 1 zur Ermittlung eines Widerstands einer Arzneimittelzubereitung/Probe 2. Die Messkammer 1 weist hierzu vorzugsweise mindestens zwei Elektroden 3 auf. Ferner ist die Messkammer 1 dazu ausgebildet, die Arzneimittelzubereitung/Probe 2 aufzunehmen und derart mit den Elektroden 3 in unmittelbaren Kontakt zu bringen, dass die Elektroden 3 voneinander durch die Arzneimittelzubereitung/Probe 2 galvanisch getrennt sind.

Im Ausführungsbeispiel gemäß Fig. 1 sind als Elektroden 3 eine obere, bewegliche Elektrode 3A und eine untere, bevorzugt ortsfeste, Elektrode 3B vorgesehen. Im Folgenden werden diese jedoch stets weiter allgemein als Elektroden 3 bezeichnet, sofern die Erläuterung nicht deren konkrete Ausgestaltung betrifft.

Im Ausführungsbeispiel gemäß Fig. 1 ist zumindest eine, vorzugsweise die in Gebrauchslage obere, Elektrode 3A bewegbar bzw. verschiebbar. Insbesondere ist vorgesehen, dass die Elektrode 3A einen schaft- bzw. bolzenartigen Führungsteil 5 aufweist. Der Führungsteil 5 ist vorzugsweise in einer, bevorzugt hülsenartigen, Führung 6 geführt. Hier sind jedoch auch andere Lösungen denkbar.

Die Elektrode 3A ist vorzugsweise mit einem Spannmittel 7 gegen die Arzneimittelzubereitung/Probe 2 verspannt. Im Darstellungsbeispiel weist das Spannmittel 7 eine Spiralfeder auf oder ist hierdurch gebildet. Alternativ oder zusätzlich kann das Spannmittel 7 jedoch auch hydraulisch, pneumatisch oder durch andere Feder- oder elastische Elemente realisiert werden. Das Spannmittel 7 ist vorzugsweise dazu vorgesehen, ein Einspannen oder Einklemmen der Arzneimittelzubereitung/Probe 2 zwischen den Elektroden 3 zu bewirken.

Im Darstellungsbeispiel bildet die Führung 6 ein Gegenlager 8 für das Spannmittel 7. Alternativ oder zusätzlich kann das Gegenlager 8 jedoch auch unabhängig von der Führung 6 realisiert sein.

Vorzugsweise ist die Elektrode 3A in der Messkammer 1 derart, insbesondere axial, verschiebbar oder bewegbar, dass zwischen den Elektroden 3 zum Einlegen der Arzneimittelzubereitung/Probe 2 ein Abstand herstellbar ist. Vorzugsweise ist hierzu die Führung 6 mit einem Gewinde 9 versehen, das vorzugsweise mit einem Gegengewinde 10 in Eingriff steht. Das Gegengewinde 10 ist vorzugsweise mit der Messkammer 1 verbunden oder einstückig hiermit gebildet. Durch Verdrehen der Führung 6 kann diese weiter in die Messkammer 1 hinein oder aus der Messkammer 1 herausgeschraubt werden, wodurch sich der Abstand zwischen den Elektroden 3 einstellen lässt. Hier sind jedoch auch andere Lösungen möglich, um das Gegenlager 8 und hiermit die Elektrode 3A zu bewegen.

Die Elektrode 3A kann einen Anschlag 11, einen Obstakel oder eine Schulter o. dgl. aufweisen, der/die die Bewegbarkeit der Elektrode 3A in Bezug auf die Messkammer 1 bzw. die Führung 6 und/oder das Gegenlager 8 begrenzt.

Eine oder mehrere der Elektroden 3 weist/weisen vorzugsweise einen Kraftmesser 12 auf, der dazu ausgebildet und angeordnet ist, eine auf die Arzneimittelzubereitung/Probe 2 wirkende Kraft zu messen. Vorzugsweise ist der Kraftmesser 12 zwischen einer der Elektroden 3 und der Messkammer 1 vorgesehen. Hier sind jedoch auch andere Lösungen möglich. Der Kraftmesser 12 kann mit einem Regelkreis und einem nicht dargestellten Aktuator dazu verwendet werden, eine vorgegebene oder vorgebbare Kraft auf die Arzneimittelzubereitung/Probe 2 zu regeln, insbesondere indem die Spannkraft des Spannmittels 7 und/oder die Position des Gegenlagers 8 bzw. die durch die Elektroden 3 auf die Arzneimittelzubereitung/Probe 2 wirkende Kraft beeinflusst wird.

Im Darstellungsbeispiel kann insbesondere die Führung 6 rotiert werden, so dass über das Gewinde 9 und das Spannmittel 7 die Kraft der Elektrode 3A auf die Arzneimittelzubereitung/Probe 2 verändert wird.

Die Messkammer 1 weist vorzugsweise ein Konditionierungsmittel 13 auf, das vorzugsweise dazu ausgebildet ist, durch Aufnahme und/oder Abgabe von Wasser den Wassergehalt bzw. die (relative) Feuchtigkeit der Atmosphäre 4 zu beeinflussen, einzustellen oder zu regeln.

Das Konditionierungsmittel 13 ist vorzugsweise in einem Halter 14 angeordnet, der vorzugsweise auf einer den Elektroden 3 abgewandten Seite der Messkammer 1 vorgesehen ist. Hier sind jedoch auch andere Lösungen möglich. Die Messkammer 1 ist vorzugsweise mehrteilig ausgebildet.

Die Messkammer 1 kann insbesondere in einen die Elektroden 3 aufweisenden Teil und einen das Konditionierungsmittel 13 bzw. die Halteeinrichtung 14 aufweisenden Teil getrennt werden. Hierzu ist im Darstellungsbeispiel vorgesehen, dass die Teile der Messkammer 1 durch eine Dichtung 15 gegeneinander im geschlossenen Zustand abgedichtet sind, wobei die Teile der Messkammer 1 entlang der Dichtung 15 voneinander trennbar sind.

Optional kann eine Trennwand 16 vorgesehen sein oder in die Messkammer 1 eingesetzt werden, um die Messkammer 1 mit verringertem, abgeschlossenen Volumen im Bereich der Elektroden 3 ohne Konditionierungsmittel 13 betreiben zu können. Die Seite der Messkammer 1 mit dem Konditionierungsmittel 13 kann in einer Variante abnehmbar sein. In diesem Fall ist es bevorzugt, dass die Trennwand 16 eine Abdeckung, ein Verschluss oder Deckel der Messkammer 1 ist oder bildet.

Die Elektroden 3 sind vorzugsweise mit oder mittels einem oder mehreren Kabeln 17 mit einer Messeinrichtung 18 elektrisch verbunden. Die Kabel 17 sind vorzugsweise geschirmt und/oder antistatisch ausgerüstet, um Störungen des Messvorgangs zu vermeiden.

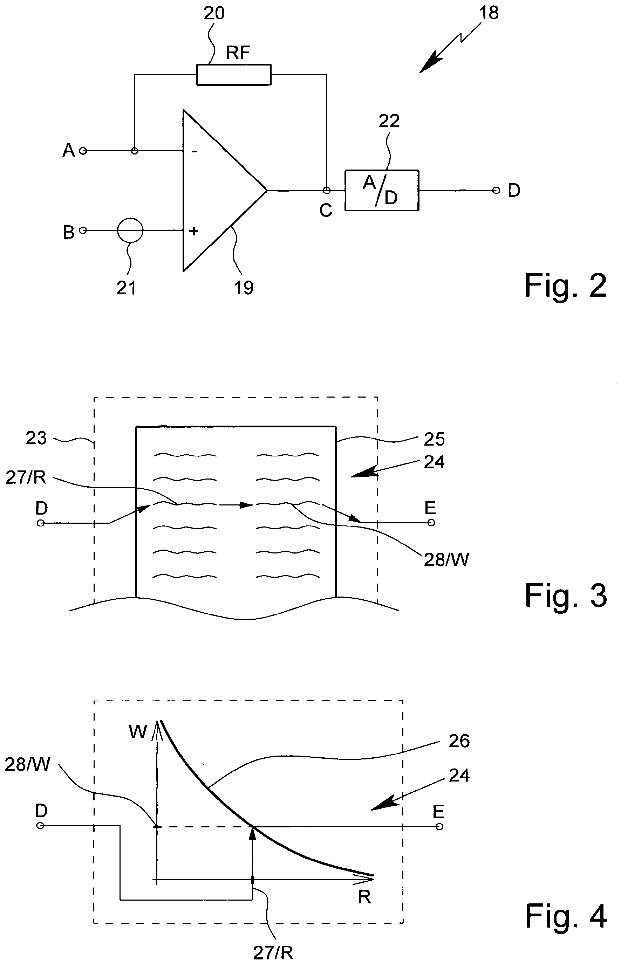

Fig. 2 zeigt eine schematische Ansicht der Messeinrichtung 18 in einer bevorzugten Variante. Alternativ sind jedoch auch andere Lösungen denkbar.

Die Messeinrichtung 18 weist in dem Beispiel gemäß Fig. 2 einen negativ rückgekoppelten Operationsverstärker 19 sowie einen Rückkopplungswiderstand 20 auf.

Zwischen den Knoten A und B, die mit den Eingängen des Operationsverstärkers 19 und/oder jeweils mit einer Elektrode 3 verbunden sind, ist vorzugsweise die Arzneimittelzubereitung/Probe 2, kontaktiert durch die Elektroden 3, vorgesehen.

Der Operationsverstärker 19 bewirkt in seiner Verschaltung gemäß Fig. 2, dass die Differenzspannung zwischen dem mit Plus-Symbol und mit Minus-Symbol gekennzeichneten Eingängen zu Null wird. Daraus folgt, dass die durch eine Spannungsquelle 21 in Reihenschaltung mit der Arzneimittelzubereitung/Probe 2 eine durch die Spannungsquelle 21 erzeugte Spannung an der Arzneimittelzubereitung/Probe 2 abfällt. Dadurch, dass die Eingänge des Operationsverstärkers 19 hochohmig sind, bewirkt der durch den Spannungsabfall über der Arzneimittelzubereitung/Probe 2 erzeugte Strom einen Spannungsabfall am Rückkopplungswiderstand 20, der dem Ausgangssignal des Operationsverstärkers 19 am Knoten C entspricht. Das Ausgangssignal wird vorzugsweise mit einem Analog-Wandler 22 digitalisiert am Ausgang D der Messeinrichtung zur Verfügung gestellt. Für die diesbezüglichen Grundlagen wird an dieser Stelle auf das Lehrbuch Halbleiter-Schaltungstechnik, U. Tietze, Ch. Schenk, Springer Verlag, 11. Auflage, 1999 verwiesen.

Die Spannung am Knoten C bzw. das sich hieraus ergebene oder korrespondierende digitale Signal am Knoten D korrespondiert vorzugsweise zum Widerstand der Arzneimittelzubereitung/Probe 2. Hierbei wird davon ausgegangen, dass aufgrund des hohen Widerstands R der Arzneimittelzubereitung/Probe 2 parasitäre Effekte durch Übergänge, Zuleitungen und dgl. vernachlässigbar sind. Alternativ oder zusätzlich können ungewollte parasitäre Effekte durch die Messeinrichtung 18 kompensierbar sein.

Das analoge Ausgangssignal am Knoten C bzw. das digitale Ausgangssignal am Knoten D der Messeinrichtung 18 entspricht dem Widerstand R oder korrespondiert zu dem Widerstand R, und wird im Sinne der vorliegenden Erfindung im Folgenden der Einfachheit und Übersichtlichkeit wegen auch "Widerstand R" oder hierzu korrespondierender Wert der Arzneimittelzubereitung/Probe 2 genannt.

Fig. 3 zeigt in einer ersten Variante eine vorschlagsgemäße Feuchtebestimmungseinrichtung 23. Die Feuchtebestimmungseinrichtung 23 weist vorzugsweise ein Zuordnungsmittel 24, im Darstellungsbeispiel eine Zuordnungstabelle 25 auf. Alternativ oder zusätzlich kann es sich auch um eine Zuordnungsfunktion 26 handeln, wie in Fig. 4 näher dargestellt.

Die Feuchtebestimmungseinrichtung 23 ist vorzugsweise dazu ausgebildet, mit dem Zuordnungsmittel 24 aus dem (ohmschen) Widerstand R bzw. dem hierzu korrespondierenden Wert einen korrespondierenden Wassergehalt W der Arzneimittelzubereitung/Probe 2 zu bestimmen. Hierbei kann ein zu dem (ohmschen) Widerstand R passender oder korrespondierender erster Wert 27 verwendet werden, um einen zu dem Widerstand R oder dem ersten Wert 27 korrespondierenden zweiten Wert 28 abzulesen oder auszulesen oder zu berechnen, der zum Wassergehalt W der Arzneimittelzubereitung/Probe 2 korrespondiert und der Einfachheit und Übersichtlichkeit wegen auch lediglich "Wassergehalt W' genannt. Der Wassergehalt W der Arzneimittelzubereitung/Probe 2 kann am Ausgang E der Feuchtebestimmungseinrichtung 23 ausgegeben werden. Insbesondere kann dieser über ein Display 29 dargestellt werden.

In einer bevorzugten Alternative wird der Wassergehalt W einer Haltbarkeitsbestimmungseinrichtung 30 übergeben, die zusammen mit Detailinformationen zu einem oder mehreren Verpackungssystemen eine Haltbarkeit der Arzneimittelzubereitung/Probe 2 mit dem bestimmten Wassergehalt W der Arzneimittelzubereitung/Probe 2 berechnet, prognostiziert und/oder simuliert. Hierbei werden vorzugsweise zusätzlich Wassereinträge und/oder Sorptions- bzw. Diffusionseigenschaften des Verpackungsmaterials und/oder der Arzneimittelzubereitung/Probe 2 berücksichtigt bzw. kombiniert.

Als Ergebnis kann am Knoten bzw. Ausgang F der Haltbarkeitsbestimmungseinrichtung 30 ein geeignetes Verpackungssystem, mehrere geeignete Verpackungssysteme, eine Haltbarkeit der Arzneimittelzubereitung/Probe 2 im in einem oder unterschiedlichen Verpackungssystemen verpackten Zustand, ein Zusatzstoffbedarf ein Trocknungsmittelbedarf und/oder ein oder mehrere korrespondierende Werte oder Indikatoren o. dgl. ausgegeben werden und/oder mit dem Display 29 angezeigt werden.

Das Zuordnungsmittel 24 wird vorzugsweise durch eine Kalibriermessung bestimmt oder ist hierdurch bestimmbar. Hierzu wird mindestens eine Arzneimittelzubereitung/Probe 2 vermessen, also der Widerstand R dieser bestimmt. Besonders bevorzugt werden mit der Messeinrichtung 18 mehrere Arzneimittelzubereitung/Proben 2 vermessen, die dieselbe Zusammensetzung, jedoch unterschiedliche Wassergehalte W aufweisen. Folglich werden durch die Messeinrichtung unterschiedliche, von dem jeweiligen Wassergehalt W abhängige ohmsche Widerstände ermittelt.

Im Folgenden wird zu der jeweiligen Arzneimittelzubereitung/Probe 2 mit einer Referenzmessmethode der tatsächliche Wassergehalt W bestimmt. Dieser tatsächliche Wassergehalt W wird dann dem korrespondierenden Widerstand R zugeordnet, wodurch das Zuordnungsmittel 24 gebildet werden kann.

Durch die Zuordnung des jeweiligen tatsächlichen Wassergehalts W zu dem Widerstand R der Arzneimittelzubereitung/Probe 2 oder hierzu korrespondierender Werte können Wertepaare gebildet werden. Diese Wertepaare, die zu einer Zuordnung eines jeweiligen Widerstands R zu dem Wassergehalt W korrespondieren, werden vorzugsweise in einer Zuordnungstabelle 25 abgelegt und/oder, insbesondere durch Interpolation, in eine Zuordnungsfunktion 26 umgewandelt.

Bei der Erzeugung des Zuordnungsmittels 24, für die Referenzmessungen und/oder im späteren Betrieb werden vorzugsweise die (jeweils) gleichen Umgebungsbedingungen, insbesondere bezüglich der Temperatur und (relativen) Feuchtigkeit der Atmosphäre 4, vorgegeben oder eingestellt. Hierdurch kann die Genauigkeit des Messverfahrens wesentlich verbessert werden.

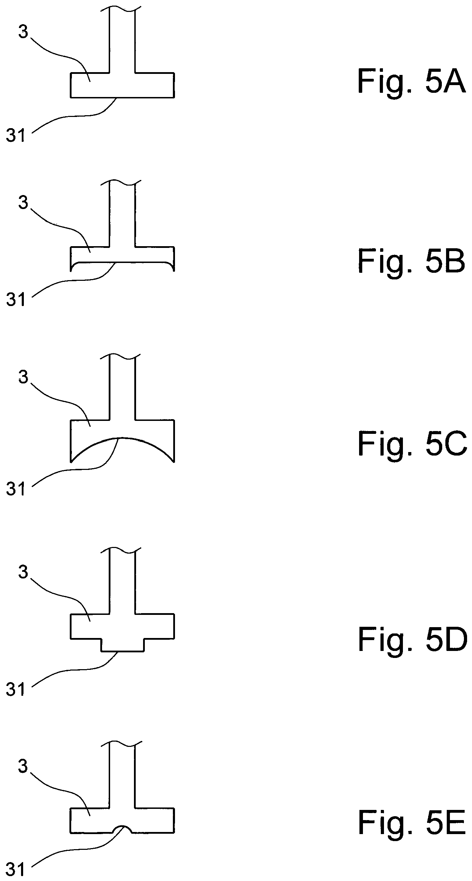

Vorzugsweise ist mindestens eine, insbesondere sind mehrere oder alle, Elektroden 3 in der Messkammer 1 austauschbar. Hierdurch kann die jeweilige Kontaktoberfläche der Elektroden 3 der Form der vorliegenden Arzneimittelzubereitung/Probe 2 angepasst sein oder angepasst werden. In den Fig. 5A bis 5E sind unterschiedliche schematische Querschnitte potentiell einsetzbarer Elektroden 3 dargestellt.

Die Elektroden 3 sind vorzugsweise im Querschnitt zumindest im Wesentlichen T- und/oder stempelförmig, können jedoch auch anders ausgebildet sein.

Die Elektrode 3 aus Fig. 5A weist eine zumindest im Wesentlichen flache bzw. ebene Kontaktoberfläche 31 auf. Die Elektrode aus Fig. 5A ist für flache Tabletten oder Tabletten mit ebenen Flachseiten besonders gut geeignet, da ein flächiger Kontakt herstellbar ist, was die Messgenauigkeit verbessert und die Wahrscheinlichkeit von kontaktbedingten Fehlern reduziert.

Die Elektrode 3 aus Fig. 5B weist eine, bevorzugt rotationssymmetrisch, leicht nach innen bzw. konkav gewölbte Kontaktoberfläche 31 auf, bei der der Radius zur Symmetrieachse oder Mittelachse zunimmt. Die Kontaktfläche 31 ist vorzugsweise komplementär zu am Rand abgerundeten Tabletten ausgebildet und liegt an diesen folglich großflächig an.

Die Elektrode 3 aus Fig. 5C hat eine mit einem zumindest im Wesentlichen konstanten Radius konkav gekrümmte, insbesondere stetig konkav gekrümmte, Kontaktoberfläche 31, die insbesondere zur Kontaktierung von linsenartigen oder linsenförmigen oder auch runden Tabletten vorteilhaft ist, da auch hier eine verhältnismäßig große Kontaktfläche 31 zwischen der Elektrode 3 und der Arzneimittelzubereitung/Probe 2 erreicht werden kann. Die Kontaktfläche 31 kann rotationssymmetrisch oder im Querschnitt oval sein.

Die Elektrode 3 aus Fig. 5D weist eine stempelartige Kontaktoberfläche 31 und/oder Kontaktfläche 31 mit einem Flächeninhalt auf, der kleiner als das 0,5-fache, vorzugsweise als das 0,25-fache des Durchmessers der zu messendender Arzneimittelzubereitung/Probe 2 ist. Die Kontaktfläche ist vorzugsweise zumindest im Wesentlichen eben und/oder flach ausgebildet. Die Elektrode 3 bietet den Vorteil einer definierten Kontaktfläche auch bei ungewöhnlichen Randkrümmungen der Arzneimittelzubereitung/Probe 2. Um dies zu erreichen, kann die Kontaktfläche auf die T-förmige Grundform aufgesetzt sein oder werden.

Die Elektrode 3 aus Fig. 5E weist eine vorzugsweise kontinuierliche und/oder längliche Aufnahme, Wölbung oder Einkerbung auf, die hohlzylinderabschnittartig gebildet sein kann, vorzugsweise mit einem Radius größer als 2 mm und/oder kleiner als 5 mm. Hierdurch können Kapseln optimal kontaktiert werden.

Allgemein sind die Elektroden 3A, 3B (jeweils) gleich ausgebildet und/oder weisen gleiche oder ähnliche Kontaktoberflächen 31 auf. Etwas anderes kann gelten, falls asymmetrisch gebildete Arzneimittelzubereitungen/Proben 2 vermessen werden sollen. Insgesamt ist es besonders bevorzugt, dass die Kontaktoberflächen 31 der Elektroden 3 komplementär zu der Darreichungsform bzw. dem äußeren Erscheinungsbild der jeweiligen Arzneimittelzubereitung/Probe 2 gebildet sind.

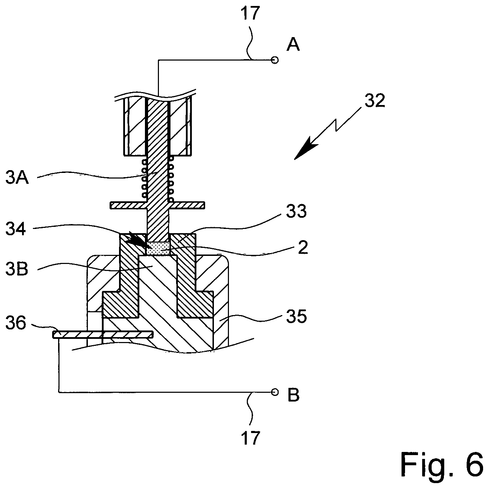

In Fig. 6 ist eine Elektrodenanordnung 32 dargestellt, bei der zusätzlich zu den Elektroden 3A, 3B eine von einem Isolator 33 begrenzte Kavität 34 zur Aufnahme der Arzneimittelzubereitung/Probe 2 in granularer Form oder Pulverform vorgesehen ist.

Vorzugsweise ist eine der Elektroden 3A in der Kavität 34 bewegbar und/oder komplementär hierzu gebildet. Insbesondere bildet die eine der Elektroden 3A mit der Kavität 34 zumindest abschnittsweise eine Zylinder-Kolben-Anordnung. Hierdurch kann erreicht werden, dass die eine der Elektroden 3A die Arzneimittelzubereitung/Probe 2 zumindest im Wesentlichen abdeckt und/oder vollflächig kontaktiert und/oder komprimiert, insbesondere durch die Spannung des Spannmittels 7.

Eine zweite oder andere der Elektroden 3B ist bei der Elektrodenanordnung 32 aus Fig. 6 im unteren Bereich angeordnet und bildet vorzugsweise zumindest teilweise den Boden der Kavität 34. Hierdurch kann eine großflächige Kontaktierung und genaue Bestimmung des Widerstands R und folglich des Wassergehalts W erreicht werden.

Die Elektrodenanordnung 32 kann optional weiter einen isolierenden Überwurf 35, insbesondere eine Überwurfmutter, zur Befestigung des Isolators 33 aufweisen. Eine Befestigung kann jedoch auch anders erfolgen.

Ferner weist die Elektrodenanordnung 32 vorzugsweise einen Kontaktstift 36 auf, über den das Kabel 17 mit der Elektrode 3B elektrisch verbunden werden kann. Die obere Elektrode 3A kann entsprechend der Lösung aus Fig. 1 oder auf andere Weise vorgespannt sein bzw. das Spannmittel 7 aufweisen (in Fig. 6 nicht dargestellt). Alternativ oder zusätzlich zu dem Spannmittel 7 kann die Elektrodenanordnung 32 auch den Kraftmesser 12 aufweisen. Hier sind jedoch ebenfalls andere Lösungen möglich.

Die Elektroden 3 der Elektrodenanordnung 32 sind vorzugsweise durch Auswechseln oder Einsetzen in die oder in der Messkammer 1 einsetzbar, wobei eine Messung oder Auswertung in entsprechender Weise wie in Zusammenhang mit Fig. 1 und 2 beschrieben erfolgen kann.

Die Messkammer 1 weist vorzugsweise eine Kammerwand 37 auf, die die Elektroden 3 und/oder die Elektrodenanordnung 32 und/oder die Atmosphäre 4 und/oder das Konditionierungsmittel 13 und/oder die Halteeinrichtung 14, vorzugsweise luftdicht, einschließt. Die Kammerwand 37 kann aus Metall oder sonstigen starren Materialien gebildet sein. Die Kammerwand 37 bildet vorzugsweise einen hermetisch abschließbaren Innenraum, der vorzugsweise mit der Atmosphäre 4 gefüllt ist. Die Kammerwand 37 ist vorzugsweise wärmeleitend, kälteleidend und/oder dazu ausgebildet, der Atmosphäre 4 Energie zuzuführen und zu entziehen. Die Messkammer 1 kann in einem Klimaschrank zur Temperatureinstellung angeordnet sein oder ein Klimaschrank kann die Kammerwand 37 zur Temperierung der Atmosphäre 4 umgeben. Die Temperatur der Atmosphäre 4 wird geregelt. Dies kann von außen durch die Kammerwand 37, aber auch durch eine direkte Temperierung der Kammerwand 37 oder der Atmosphäre 4 erfolgen.

Zur Sicherstellung der Produktqualität von feuchteempfindlichen Produkten sind möglichst dichte Verpackungssysteme unter Zugabe von meist granularen Trockenmitteln bekannt.

Eine Messung der Feuchtevorbelastung mit evaluierten Standard-Methoden erreicht bestenfalls eine Genauigkeit von ±0,2%m/m, sie ist außerdem zeitaufwendig, störungsanfällig und zerstörend.

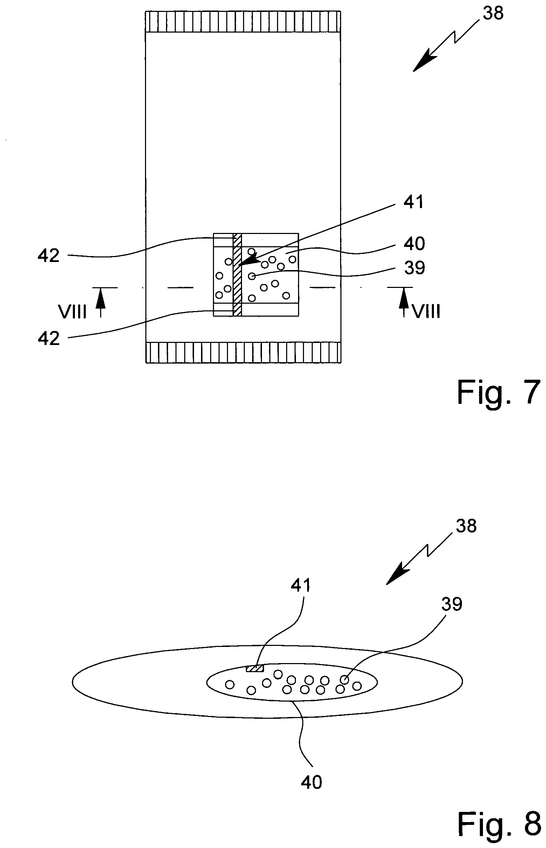

Ein Aspekt, der nicht Gegenstand der Erfindung ist, betrifft eine bevorzugt beutelartige Verpackung 40 (vgl. Fig. 7) für bevorzugt granulare Stoffe wie Trockenmittel 39, wobei das die Verpackung bildende Material oder ein in die Verpackung eingebrachtes Material einen feuchteabhängigen Widerstand aufweist und eine Widerstandseinrichtung 41 bildet.

Die Trockenmittel (typischerweise Silikagel, Molekularsieb, Bentonit oder Mischungen derselben) werden meist in Verpackungen 40 für Trockenmittel, insbesondere Sachets aus HDPE-Fasern bzw. PE-Fasern verwendet. Diese Verpackungen 40 sind vorzugsweise zumindest teilweise wasserdampfdurchlässig, um eine Trennung zwischen Trockenmittel 39 und Atmosphäre 4 herzustellen, gleichzeitig jedoch die Sorption von Wasser durch das Trockenmittel 39 zu ermöglichen.

Die Widerstandseinrichtung 41 ist vorzugsweise an unterschiedlichen Kontaktstellen derart kontaktierbar, dass zwischen diesen Kontaktstellen 42 der Widerstand R der Widerstandseinrichtung 41 gemessen werden kann. Im Folgenden kann mit dem Widerstand R auf die Feuchtigkeit/den Wassergehalt W der Widerstandseinrichtung 41 und, vorzugsweise, mittelbar über diesen Widerstand R auf die Feuchtigkeit / den Wassergehalt W des Inhalts der Verpackung 40, vorzugsweise des Trockenmittels 39, geschlossen werden.

Die Widerstandseinrichtung 41 wird hierzu vorzugsweise ins thermodynamische Gleichgewicht mit dem Trockenmittel / dem Inhalt der Verpackung 40 gebracht. Auf diese Weise kann über den Widerstand R der Widerstandseinrichtung 41 die Feuchtigkeit der Widerstandseinrichtung 41 und mittelbar die Feuchtigkeit bzw. Beladung des Trockenmittel / Inhalts der Verpackung 40 ermittelt werden. Hierzu können Zuordnungsmittel 24 wie oben beschrieben erzeugt bzw. verwendet werden.

Es ist bevorzugt, dass die Widerstandseinrichtung 41 mit dem feuchteabhängigen Widerstand R an unterschiedlichen Seiten der Verpackung 40 kontaktiert und daran die elektrische Leitfähigkeit bzw. der Widerstand R gemessen wird.

Weitere Schritte, insbesondere Kalibrierung und Messung, können wie zuvor beschrieben erfolgen, vorzugsweise wobei die Kalibrierung mit der Widerstandseinrichtung 41 erfolgt.

Besonders bevorzugt wird als Material mit feuchteabhängigen Widerstand R bzw. als Widerstandseinrichtung 41 ein Widerstandsstreifen mit feuchteabhängigem Widerstand R bzw. eine Folie mit einem feuchteabhängigen Widerstand R, vorzugsweise als Beilaufstreifen, in die Verpackung 40 eingebracht, insbesondere eingeschweißt. Vorzugsweise erstreckt sich die Widerstandseinrichtung 41 längs entlang und/oder innerhalb der Verpackung 40 zwischen unterschiedlichen, bevorzugt gegenüberliegenden Seiten der Verpackung 40.

Vorzugsweise wird als Widerstandseinrichtung 41 ein Material verwendet, das in Abhängigkeit von der Wassersättigung eine Differenz des Widerstands R in der verwendeten Konfiguration mindestens um einen Faktor 10, 100 oder 1000 aufweist.

Vorzugsweise wird oPA-Folie (oPA = orientiertes Polyamid) als Widerstandseinrichtung 41, insbesondere in Form eines Beilaufstreifens, eingesetzt.

Dies ermöglicht die Nutzung der sehr starken Feuchteabhängigkeit des el. Widerstandes von oPA-Folie mit einer Veränderung um bis zu Faktor 10000 in üblicherweise untersuchten Feuchtebereichen. Damit kann gegenüber der Leitfähigkeitsmessung an herkömmlichen Trockenmittelbeuteln (beispielsweise aus GDT) eine Verbesserung um ca. Faktor 50 erzielt werden. Diese Methode kann auch zur Messung des Feuchtegehalts bzw. Wassergehalt W sonstiger loser Komponenten bzw. Proben 2 eingesetzt werden.

Der Einsatz eines Beilaufstreifens erfolgt besonderes bevorzugt bei der Herstellung von Verpackungen 40 aus schlauchartigem Endlosmaterial, wobei der Beilaufstreifen bereits im Endlosmaterial vorgesehen oder bei Verschließen und Abtrennen von beutelartigen Segmenten eingebracht, insbesondere an- und/oder eingeschweißt werden kann.

Es ist ferner bevorzugt, Verpackungen 40 in Form von Sachet-Hüllen für Trockenmittel aus einem Material mit starker Feuchteabhängigkeit des el. Widerstandes (z.B. oPA) anzufertigen. Alternativ oder zusätzlich kann ein solches Material in das Gewebe von Sachet-Hüllen in Anteilen eingewoben werden. Dies eröffnet den Vorteil, direkt am Hüllenmaterial der Verpackung 40 messen zu können, was den Aufwand / die Kosten, einen Beilaufstreifen mit zu verarbeiten, erspart.

Es ist bevorzugt, Verpackungen 40 von Bulkware (z.B. Pouches, Kreuzbodensäcke) mit einer Widerstandseinrichtung 41, insbesondere in Form eines Beilaufstreifens o. dgl., zu versehen, vorzugsweise auf einer Innenseite. Dies eröffnet die Möglichkeit, die r.F. und damit den Feuchtegehalt bzw. Wassergehalt W des Inhalts sehr genau zu messen, ohne die Verpackung zu öffnen bzw. zu beschädigen. Dies bietet den Vorteil einer hohen Genauigkeit der Feuchtegehaltsmessung und Senkung des Risikos, den Verpackungsinhalt durch den Messprozess (Öffnen bzw. perforieren) nachhaltig zu beschädigen.

Es wird vorgeschlagen, allgemein Verpackungen 40, insbesondere Trockenmittel-Container, (z.B. Flaschenverschlüsse, Trockenmittel bzw. Sorbentien enthaltende Kapseln) mit einer Widerstandseinrichtung 41, insbesondere in Form des Beilaufstreifens, zu versehen und dadurch auch Feuchtegehaltsmessungen bzw. Wassergehaltmessungen zu ermöglichen.

Beispielsweise werden Ca-basierte Trockenmittelkarten aus Faserstoff eingesetzt. Zu einer Verbesserten Messung deren Feuchtegehalts bzw. Wassergehalts W wird vorgeschlagen, sie mit einer Widerstandseinrichtung 41 wie mit dem Beilaufstreifen bzw. mit einer Folie zu bekleben oder zu verschweißen, welche wie beschrieben zur Feuchtegehaltsmessung der Karte verwendet werden kann.

Bevorzugt sind ferner Anwendungen bei Vielschicht-Verpackungen, z.B. beim so genannten Standard-Wannen-Blister. In einer Variante kann die Feuchte / der Wassergehalt W in der Verpackung 40 über die Leitfähigkeit bzw. den Widerstand R der Wannen-Folie gemessen werden. Die Wannenfolie ist hierbei als Widerstandseinrichtung 41 ausgebildet oder fungiert als solche.

Gemäß einem Aspekt der vorliegenden Erfindung wird das vorschlagsgemäße Verfahren auch für Adsorber für andere volatile Stoffe eingesetzt (z.B. für Sauerstoff, geruchsbildende Volatile, Lösungsmittel). Deren Sorptionskapazität für volatile Stoffe ist meist auch abhängig von der Vorbeladung mit Wasser. Diese Adsorber (z.B. Eisenoxid-, Aktivkohle- oder Zeolith-basierte Sorbentien) können ebenfalls in granularer Form in Sachets verpackt verwendet werden. Ferner ist die Methode der Feuchtegehaltsmessung bzw. Wassergehaltsmessung auch hier zur Messung der Wasservorbeladung einsetzbar und damit ihre Sorptionskapazität für andere Volatile kontrollierbar.

Gemäß einem weiteren Aspekt, der jedoch nicht Gegenstand der vorliegenden Erfindung ist, wird die elektrische Leitfähigkeit bzw. der Widerstand R zur Voruntersuchungen hinsichtlich der Probenqualität von pharmazeutischen Wirkstoffen und/oder Hilfsstoffen genutzt.

Eine zentrale Idee der vorliegenden Erfindung ist eine in ihrer Genauigkeit verbesserte, schnelle, kostengünstige, mobil einsetzbare und zerstörungsfreie Methode zur Messung des Feuchtegehalts in festen Formulierungen von pharmazeutischen Produkten, deren Verpackungen, Packmitteln, Packmittelkomponenten, insbesondere Trockenmitteln und Folien, Verpackungen und Devices, insbesondere Proben hiervon, mit Hilfe der elektrischen Leifähigkeit bzw. deren elektrischen Widerstand R.

Der elektrische Widerstand R ist als Kehrwert des elektrischen Leitwerts definiert, weshalb die Begriffe teils entsprechend oder synonym verwendet werden können bzw. austauschbar sind.

Das bevorzugte Messprinzip beruht darauf, dass die elektrische Leitfähigkeit einer Probe 2 feuchteabhängig ist. Gemäß einem Aspekt wird zwischen einem Widerstand R bzw. einer Leitfähigkeit differenziert bzw. werden ein oder mehrere Widerstände R oder Leitfähigkeiten berücksichtigt, die auf Wasserionen selbst basiert und einen Beitrag, der durch die Wasserionen mobilisierte Ionen aus dem Festkörper.

Es wird vorzugsweise angenommen, dass es einen mathematisch eineindeutigen, d.h. bijektiven Zusammenhang zwischen elektrischer Leitfähigkeit und dem Feuchtegehalt gibt. Dieser Zusammenhang wird vorzugsweise als Zuordnungsmittel 24 oder zu dessen Bestimmung verwendet

Das vorschlagsgemäße Verfahren basiert vorzugsweise auf einer oder mehreren Gleichstrommessung(en) (DC) des elektrischen Widerstands R der Probe 2. Vorzugsweise wird aus gemessener Spannung und gemessenem oder vorgegebenem Strom der Ohm'sche Widerstand R der Probe 2 berechnet.

Die Messeinheit bzw. die Messkammer 1 selbst weist vorzugsweise ein sehr präzises Elektrometer auf. Es ist bevorzugt, dass mittels des Elektrometers über die Ausgabe einer konstanten Hochspannung und/oder als Konstantstromquelle genutzt wird.

Die Probe 2 ist vorzugsweise hochohmig, beispielsweise mit mehr als 1 Megaohm, und/oder ein Halbleiter. Die Probe 2 kann auch einen Widerstand R im mehrstelligen Megaohmbereich haben, beispielsweise mehr als 10⁸, 10⁹ oder 10¹⁰, teils bis zu 10¹³ oder 10¹⁴ Ohm bis hin zu 10¹⁵ oder sogar 10¹⁶ Ohm aufweisen. Diese Wiederstände R werden gemessen, um Rückschlüsse auf die Feuchtigkeit bzw. den Wassergehalt W ziehen zu können.

Gemäß einem Aspekt der vorliegenden Erfindung weist die Messkammer 1 zwischen voneinander isolierten bzw. räumlich mindestens 5 mm getrennten Elektroden 3 einen Widerstand von mehr als 10¹⁶ Ohm, vorzugsweise mindestens 10¹⁷ Ohm auf. Die Messung kann bei einer mit Luft befüllten Messkammer 1, bei 25°C, Normaldruck von 1013 hPa und/oder einer relativen Luftfeuchtigkeit von maximal 40%, vorzugsweise weniger als 30%, insbesondere weniger als 20% oder 10 % erfolgen. Alternativ erfolgt die Messung bei technisch trockener, stickstoffbefüllter und/oder evakuierter Messkammer 1. Die zur Erzielung dieses Widerstands getroffenen Maßnahmen, insbesondere hochisolierte elektrische Zuführungen und Durchführungen zu den Elektroden 3 ermöglichen in vorteilhafter Weise eine ausreichend genaue Messung auch sehr hoher Probenwiderstände wie zuvor angegeben.

Die hohen Widerstandswerte bedingen sehr geringe Messströme (vorzugsweise weniger als 1000 pA, insbesondere weniger als 500 pA oder 200 pA). Dadurch ist es bevorzugt, Kriechströme und kapazitive Effekte an der Apparatur zu vermeiden oder zu reduzieren, letztere v.a. auch an den Proben 2. Vorteilhaft sind ein Vibrationsschutz und/oder eine elektrische oder elektromagnetische Abschirmung in dem Messaufbau und/oder spezifische Probenhalter für Messungen an unterschiedlichen Formen fester Proben 2.

Die Probe 2 wird vorzugsweise in einer Form verwendet, in der diese verpackt wird oder werden soll. Insbesondere handelt es sich um eine Arzneimittelzubereitung in der jeweiligen Darreichungsform, wie eine Tablette.

Die Probe 2 wird vorzugsweise zerstörungsfrei charakterisiert. Insbesondere ist vorgesehen, dass die Leitfähigkeit bzw. der elektrische Widerstand R der Probe bestimmt wird, ohne die Integrität der Probe 2 zu beeinträchtigen. Die Charakterisierung des Widerstands R bzw. des Leitwerts erfolgt also bevorzugt zerstörungsfrei.

Vorzugsweise erfolgt eine Kalibrierung. Es erfolgt vorzugsweise eine Vorkonditionierung der Proben 2 für die Kalibrierung. Hierbei ist bevorzugt, die Proben 2 hinsichtlich ihres Feuchtegehalts bzw. Wassergehalts W vorzukonditionieren. Hierzu werden vorzugsweise sowohl Feuchte (Wassergehalt W) als auch Temperatur der Probe 2 vorgegeben, beispielsweise mit Hilfe eines Klimaschranks. Alternativ oder zusätzlich kann die Probe 2 in einem dicht verschlossenen Behälter, beispielsweise einem Exsikkator, über einer gesättigten Salzlösung bei einer bestimmten relativen Feuchtigkeit in bei einer konstanten und definierten Temperatur gelagert werden.

Zur Kalibrierung wird die Probe 2 vorzugsweise bis zur thermodynamischen Relaxation (vollständige Angleichung an die Temperatur und insbesondere an die Gleichgewichtsfeuchte) konditioniert. Die Relaxationszeiten können aus DVS-Messungen, alternativ gravimetrisch über hinreichend empfindliche Analysenwaagen, und/öder durch eine hochempfindliche Messung der relativen Feuchtigkeit, beispielsweise mit Hilfe der µ-GC ermittelt werden.

Im eigentlich bestimmungsgemäßen Betrieb, d.h. bei der Messung von Proben unbekannter Feuchte, ist vorzugsweise sicherzustellen, dass diese Proben sich im thermisch relaxierten Zustand, d.h. im thermodynamischen Gleichgewicht mit ihrer Umgebung sowohl hinsichtlich der Temperatur, als auch hinsichtlich der Gleichgewichtsfeuchte befinden, ohne dass dabei die Probe umkonditioniert wird.

Es wird vorzugsweise davon ausgegangen bzw. dafür Sorge getragen, dass beim Probenzug thermodynamisches Gleichgewicht gegeben ist, d.h. dass die Probe sich bereits lange genug in dem Klima befindet und sich kurz vor der Entnahme innerhalb der materialspezifischen Relaxationszeit weder Temperatur noch relative Feuchtigkeit geändert haben. Für den Transport zur Messung bzw. Zwischenlagerung sollte die Temperatur nur so weit geändert werden, dass Phasenübergänge und alle weiteren sorptionsrelevanten Effekte sicher ausgeschlossen sind.

Eine Veränderung des Feuchtegehalts der Probe 2 vor ihrer Charakterisierung bzw. vor der Messung Ihres Widerstands R wird beispielsweise durch eine zumindest im Wesentlichen wasserdampfdichte Verpackung (Al-Pouches, Stickpacks, Glasflaschen mit speziellen Verschlüssen - beispielsweise mit teflonbasierten Dichtungen - und/oder eine ausreichend hohe Probenmenge und einen so geringen umgebenden Gasraum/Atmosphäre 4, dass eine Umkonditionierung durch Feuchteaustausch verschwindend gering Bleibt, verhindert. Das die Probe 2 innerhalb einer solchen Verpackung umgebene Volumen (Atmosphäre 4) ist beispielsweise geringer als 20 %, 10 %, vorzugsweise 5 %, insbesondere 3 %, des Volumens der Probe 2.

Für den Fall, dass unklar ist, ob die Probe 2 sich im thermodynamischen Gleichgewicht befindet bzw. wenn eine bestimmte Probe 2 unabhängig von ihrem thermodynamischen Gleichgewichtszustand so schnell wie möglich mit Hilfe der elektrischen Leitfähigkeit untersucht werden soll, sind folgende Möglichkeiten bevorzugt:
In einer Variante wird die Probe 2 sehr dicht und mit geringem Luftraum (geringem Atmosphärevolumen) dampfdiffusionsdicht verpackt und/oder bei einer kontrollierten Temperatur die Relaxationszeiten für Temperatur- und Feuchteausgleich bei dieser Temperatur abgewartet.

Alternativ oder zusätzlich wird dieser Prozess durch Aufheizen in der dichten und hinreichend kleinen Verpackung beschleunigt, vorzugsweise wobei die bei der Arbeitstemperatur materialspezifische Relaxationszeit abgewartet und die Probe 2 anschließend wieder auf Messtemperatur abgekühlt wird.

In einer besonders bevorzugten Variante wird von der relaxierenden Probe 2 zu mehreren unterschiedlichen Zeitpunkten bzw. in bestimmten Zeitintervallen, beispielsweise stündlich, der Widerstand R bzw. Leitwert gemessen (ähnlich wie bei Blutzucker- oder Alkoholtests) und auf den Leitfähigkeitswert bzw. Widerstand R im relaxierten Zustand zu extrapoliert.

Damit die Konditionierung auch während der Messung sichergestellt ist, wird die Messkammer 1 klimatisiert, beispielsweise auf 25°C. Dies ist für die Messgenauigkeit Ausschlaggebend, da sich mit der Temperatur der Feuchtegehalt bzw. Wassergehalt W der Probe 2 ändern könnte (Grund: Temperaturabhängigkeit der Sorptionskapazität), und/oder der elektrische Widerstand R sich ändert (Grund: thermische Aktivierung des Leitfähigkeitsmechanismus.

Die Konditionierung kann beispielsweise in einem Klimaschrank realisiert werden. Damit geht, wie auch bei der Kalibrierung, die Genauigkeit des Klimaschranks in diejenige der Messung mit ein. Bei Versuchen hat sich herausgestellt, dass die Messgenauigkeit mit der vorschlagsgemäßen Messkammer 1 so hoch ist, dass die Genauigkeit des Klimaschranks und die der Referernzfeuchtemessmethode (DVS) und ggfs. des Elektrometers die derzeit begrenzenden Faktoren für die Genauigkeit der hier beschriebenen Feuchtemessmethode sind.

Für die Genauigkeit sowie für die Standzeit der Apparatur während der Kalibrierung und im Messbetrieb ist ein stabiler Feuchtegehalt bzw. Wassergehalt W der Probe 2 und damit eine kontrollierte und stabile relative Feuchte in der Probenkammer / der Atmosphäre 4 entscheidend.

Vorzugsweise erfolgen alle Messungen in einer abgedichteten Messkammer 1. Die Messkammer 1 weist vorzugsweise eine temperaturregulierende Einrichtung wie eine Kühleinrichtung wie einen Kompressor, eine Heizung wie eine Widerstandsheizung und/oder ein Peltierelement, und, vorzugsweise, einen Temperatursensor mit Regelung zur Steuerung der Kühleinrichtung und der Heizung auf. Zusätzlich ist die Messkammer 1 zur Einstellung einer bestimmten (relativen) Feuchtigkeit der in der Messkammer 1 befindlichen Atmosphäre 4 ausgebildet.

Vorzugsweise wird, jedenfalls für die Kalibrierung, ein konstantes Klima mit einer gesättigten Salzlösung und/oder vorkonditioniertem Trockenmittel (beispielsweise Silikagel) sichergestellt. Die gesättigte Salzlösung und das vorkonditionierte Trockenmittel werden hierzu vorzugsweise gemeinsam innerhalb der Messkammer 1 angeordnet, so dass diese mit der Atmosphäre 4 innerhalb der Messkammer 1 unmittelbar in Kontakt stehen. Diese Kombination verbindet mehrere Vorteile: sie stellt die Konditionierung der Probe 2 auch über einen längeren Messzeitraum sicher, das Salz stellt dabei eine hohe Sorptionskapazität sicher (ist aber träge), das Trocknungsmittel/Silikagel hingegen ist sehr schnell in der Feuchteregulierung der Kammer auf den Zielwert. Das beschleunigt die Messzeit durch Verkürzen der Relaxationszeit nach Beladen der Messkammer 1. Die Relaxationszeit kann mit einem Stechfühler überprüft werden.

Die Salzlösung und das vorkonditionierte Trockenmittel können jedoch auch einzeln verwendet bzw. in der Messkammer 1 angeordnet werden.

Im Messbetrieb kann eine Probe 2 unbekannter Gleichgewichtsfeuchte untersucht werden, ohne dass die vorangehend für die Kalibrierung beschriebene Stabilisierung der Messung erfolgt. Hierzu kann zur zusätzlichen Stabilisierung der Messung (d.h. zur Sicherstellung der ursprünglichen Probenfeuchte und Minimierung des Messfehlers) über die Abdichtung der Messkammer 1 hinaus der Luftraum der die Probe unmittelbar in kontakt stehenden Atmosphäre 4 durch eine Metalleinlage oder sonstige Abtrennung reduziert werden. Diese Reduktion der Luftmenge bzw. des Volumens der die Probe 2 umgebenden Atmosphäre 4 (die gemeinsam mit der Probe 2 im Inneren der Messkammer 1 eingeschlossen ist) minimiert den Feuchteumsatz zwischen Probe 2 und Luftraum / Atmosphäre. Alternativ kann eine verkleinerte Messkammer 1 eingesetzt werden. Darüber hinaus kann eine Umkonditionierung der Probe 2 durch Konstruktion der Messkammer 1 und/oder ihrer Teile mit Materialien mit sehr geringen Sorptionskapazitäten minimiert werden.

Eine weitere besondere Herausforderung ist die Minimierung von Kriechströmen außerhalb der Messkammer 1, beispielsweise an den Zuleitungen, und innerhalb der Messkammer 1. Vorzugsweise werden Messleitungen bzw. Kabel 17 verwendet, die durch hochisolierende Materialien (insbesondere der Kabelhüllen) Kriechströme minimieren. Ferner werden bei der Messung mit der Probe in Kontakt tretende Teile vorzugsweise entfettet. Darüber hinaus werden insbesondere Vibrationen in den Zuleitungen bzw. Kabeln 17 Unterbunden, vorzugsweise durch eine Befestigung gegen Bewegung gesichert. Die Zuleitungen bzw. Kabel 17 werden bevorzugt gegen Einkopplung von elektrischen, magnetischen und/oder elektromagnetischen Störungen abgeschirmt. Ferner ist das Elektrometer vorzugsweise dazu ausgebildet, Wechselsignalanteile zu verwerfen oder auszufiltern.

Wechselsignale sind vorzugsweise Signale, die Frequenzanteile von mehreren, vorzugsweise mehreren hundert Hetz aufweisen oder hieraus bestehen. Gleichstrom oder Gleichspannung liegt vorzugsweise auch dann vor, wenn Ströme oder Spannungen rampenartig oder auf sonstige Weise langsam geändert werden, beispielsweise mit einer Periode von mehr als 100 ms, vorzugsweise 200 ms oder 500 ms, insbesondere einer Periode von einer oder mehreren Sekunden, vorzugsweise mehr als 2 oder 5 Sekunden, insbesondere 8 bis 15 Sekunden. Es kann also ausreichen, wenn der Gleichstrom bzw. die Gleichspannung über die Dauer einer solchen Periode vorliegt.

Die Probenkontakte / Elektroden 3 können aus einem leitenden, in der Form anpassungsfähigen Material gebildet sein oder solches aufweisen, beispielsweise leitfähiger Gummi, Kautschuk, Metallbürsten, Metallwolle und/oder mit einem leitfähigen Material beschichtete Faserstoffe. Dazu kann ein mit einer leitfähigen Schicht versehender, in der Form anpassungsfähiger, weicher, flexibler und/oder gummiartiger Stempel, insbesondere silberbeschichtet, verwendet werden. In einer weiteren Ausführung können Kontakte der Elektroden 3 aus den bei der Tablettierung verwendeten Stempelwerkzeugen oder ähnlich geformt hergestellt sein.

Eine weitere Herausforderung bei den Messungen sowie Auswertungen sind kapazitive Effekte in der Messapparatur. Die Proben selbst sind in der Regel Halbleiter mit sehr geringer Leitfähigkeit. Eine der Folgen daraus ist, dass eine kapazitive Aufladung der Proben unvermeidbar ist. Das bedeutet vereinfacht, dass vom Gesamtstrom, welcher in eine Probe hineinfließt, ein Teil in der Probe akkumuliert wird (kapazitiver Anteil) und ein anderer Teil auf der anderen Seite der Probe hinausfließt. Lediglich dieser Teil des Stroms charakterisiert tatsächlich die Leitfähigkeit der Probe. Um möglichst genau die Leitfähigkeit selbst zu messen, wird vorzugsweise auf die van-der-Pauw-Methode zurückgegriffen. Sie beruht u.a. auf einer speziellen Kontrolle der Potenziale und einer sukzessiven Umpolung des DC-Potenzials zur Eliminierung der kapazitiven Anteile.

Zur Optimierung der Messungen können:
- die Anzahl der vor der eigentlichen Messung durchzuführenden Umpolungen ermittelt werden, insbesondere zur Eliminierung von Einflüssen statischer Aufladungen auf die folgende Messung;
- die Relaxationszeiten ermittelt werden; und/oder
- die Anzahl der zur Auswertung herangezogenen Umpolungen ermittelt werden.

Gemäß einem weiteren Aspekt der vorliegenden Erfindung werden mehrere Proben 2 auf eine bestimmte Feuchtestufe, auf einen bestimmten gleichen Feuchte- oder Wassergehalt W konditioniert. Dies erfolgt insbesondere zur Kalibrierung, vor der Kalibrierung oder die Kalibrierung erfolgt mit entsprechend vorkonditionierten Proben 2, von denen folglich die bestimmte Feuchtestufe, der Feuchte- oder Wassergehalt W sehr genau bekannt oder bestimmbar ist.

Die Vorkonditionierung und/oder Bestimmung der Feuchtestufe, des Feuchte- oder Wassergehalts W erfolgt vorzugsweise mit einer sog. DVS-Waage bzw. einem DVS-Multisampler. Hierbei steht "DVS" vorzugsweise für das Arbeitsprinzip der Dynamischen Wasserdampfsorption.

Daraufhin werden eine dieser Proben einer Sorptionsmessung bzw. Feuchte- oder Wassgergehaltsbestimmung und eine andere der Proben der vorschlagsgemäßen Leitfähigkeitsmessung zugeführt.

Gemäß einem auch unabhängig realisierbaren Aspekt der vorliegenden Erfindung weist die Messkammer 1 eine DVS-Waage auf, ist in der oder als Messkammer eine DVS-Waage vorgesehen, bildet die Messkammer 1 eine DVS-Waage oder einen Teil dieser und/oder arbeitet die Messkammer 1, insbesondere zum Vorkonditionieren, nach dem Prinzip einer DVS-Waage.

Weiter bevorzugt ist eine Kombination der Verwendung einer DVS-Waage als sehr genaue Methode zur Messung der Wassersorption mit einer Messung der r.F. (relativen Feuchtigkeit) der die Probe 2 umgebenden Atmosphäre 4 mit dem Prinzip der so genannten Cavity-Ring-Down-Spectroscopy (CRDS). Dabei kann der tatsächliche Feuchtestrom (r.F.), welcher die Probe umspült, bzw. die r.F. der die Probe 2 umspülenden Atmosphäre 4 durch die CRDS-Apparatur geleitet werden. Die CRDS-Apparatur kann die Feuchtekonzentrationen in einem Gasstrom messen. Dadurch kann die Genauigkeit in der r.F. erhöht werden. Alternativ oder zusätzlich zu CRDS kann die Schwingquarz-Methode zur Bestimmung der r.F. mit der DVS-Methode kombiniert werden. Die genauere Bestimmung der r.F ermöglicht es, die Grenze der Genauigkeit der vorschlagsgemäßen Leitfähigkeitsmethode weiter zu verbessern.

Ergänzend wird verwiesen auf Richard A. Storey, Ingvar Ymen: "Solid State Characterization of Pharmaceuticals", John Wiley & Sons Ltd, 2011 sowie G. Berden, R, Engelen: "Cavity Ring-Down Spectroscopy Techniques and Applications", John Wiley and Sons, Inc. 2009.

Vorzugsweise erfolgt eine Zuordnung von stabilitätsrelevanten Parametern (beispielsweise Zersetzung, Dissolution, Bruchkraft (inkl. Brüchigkeit bzw. Klebrigkeit), Aussehen, physikalisch-chemische Phaseninstabilitäten) zu dem Feuchtegehalt bzw. der hierzu korrespondierenden Leitfähigkeit oder dem hierzu korrespondierenden Widerstand W der Probe 2. Diese Zuordnung ist vorteilhaft insbesondere:
a) Beim Setzen von Spezifikationen: sehr genau gemessene Stabilitätsdaten (beispielsweise von Zersetzung, Dissolution, Zerfall, etc.) können viel genauer gemessenen Feuchtegehaltswerten / Wassergehalten W zugeordnet werden. Die erhöhte Genauigkeit in der Feuchtegehaltsmessung erhöht damit den Handlungsspielraum für das Verpackungskonzept und damit für die Vermarktung des Produkts (Probe 2). Dies gilt für alle Produkte mit Feuchtespezifikation, jedoch insbesondere für feuchteempfindliche Produkte.
b) Beim Überprüfen des Feuchtegehalts, beispielsweise in der Qualitätssicherung, Zulassung, Ongoing Stability, Entwicklung, etc. Dies erhöht wiederum den Handlungsspielraum für Verpackungskonzept und Vermarktung.
c) Bei der Überprüfung/Freigabe der Bulkware (Probe 2) zur Verpackung. Dies erhöht die Sicherheit des Produkts (verpackt Probe 2), führt zu einer Reduzierung von zu verwerfender Bulkware (Probe 2) und spart damit Ressourcen und Kosten.
d) In besonders komplexen Fällen kann es vorkommen, dass ein Handlungsspielraum für bestimmte Verpackungskonzepte erst durch eine Erhöhung der Genauigkeit der Feuchtegehaltsmessmethoden eröffnet wird. Dies gilt insbesondere für besonders feuchteempfindliche Produkte und/oder für die Vermarktung in Klimazonen mit erhöhter Feuchte und Temperatur und/oder, je nach Medikationsschema, bei sehr herausfordernden In-Use-Bedingungen. Bei derartig komplexen Konfigurationen kann es vorkommen, dass nach derzeitigem Stand der Technik Flaschen mit einer Maximalmenge von beispielsweise 7,8g Trockenmittel die Überschreitung des maximal zulässigen Feuchtegehalts nicht verhindern könnten.

Die vorschlagsgemäße Messmethode für den Feuchtegehalt ermöglicht die Optimierung der Anforderungen an die Dichtigkeit von Packmitteln, von Verpackungskonzepten, sowie eine Optimierung von Trockenmittelmengen, ermöglicht eine genauere Kenntnis über Sicherheitsreserven im Verpackungskonzept und das Haushalten damit in der QS (beispielsweise Definition der maximalen Feuchtevorbeladung bzw. minimalen Restsorptionskapazität von Trockenmitteln) sowie in der Prozesskette (beispielsweise maximale Maschinenstillstandszeiten, insbesondere in denen die Probe 2 außerplanmäßig eine länger als übliche Zeit einer Umgebung ausgesetzt ist, aus der die Probe 2 Wasser aufnehmen kann). Damit könnte die bisher u.a. zur Kompensation von Messungenauigkeiten praktizierte Überdimensionierung von Verpackungskonzepten und Sicherheitsreserven in Prozessen reduziert werden. Dies führt zu sichereren Produkten und gleichzeitig kosteneffizienteren Verpackungskonzepten sowie Prozessen.

Die Messzeit für die Leitfähigkeit beträgt vorzugsweise wenige Minuten pro Probe 2. Zusammen mit der Genauigkeit ergibt sich ein Vorteil gegenüber Standard-Methoden und sogar gegenüber der in ihrer Genauigkeit als bisheriger "Goldstandard" geltenden Labor-Referenzmethode der DVS Sorptionsmessung. Dort dauert eine Feuchtegehaltsmessung zwischen 30 Minuten und mehreren Monaten, je nach Produkt / Probe 2.

In Vorteilhafter Weise ist die Messung zerstörungsfrei. Die gleiche Probe 2 kann weiteren Untersuchungsmethoden (beispielsweise Zersetzung, Dissolution, Bruchkraft, Strukturuntersuchungen, IR, Raman, Mikroskopie, NMR, CT und/oder Untersuchung von Phasenübergängen) zugeführt werden. Durch die Kombination der Ergebnisse ergibt sich in vielen Fällen eine verbesserte Aussagekraft, insbesondere durch eine viel genauere Zuordnung von stabilitätsrelevanten Parametern und Feuchtegehalt. Bei besonders kostspieligen Produkten kann die Probe 2 sogar der ursprünglichen Verwendung wieder zugeführt werden.

Im Gegensatz zur hinsichtlich ihrer Genauigkeit einzig vergleichbaren Referenzmethode (DVS) ist die Leitfähigkeitsmethode mobil einsetzbar. Dies eröffnet ihren Einsatz in der QS während der Prozesskette.

Die besondere Kombination aus Schnelligkeit, Zerstörungsfreiheit und Mobilität der Leitfähigkeitsmessung eröffnet ihren Einsatz in zahlreichen Prozessschritten, beispielsweise bei der Bulkwarenproduktion, Stichprobenuntersuchungen bei deren Lagerung, Freigabe zur Produktion, insbesondere aber bei Maschinenstillstand und/oder bei der Zwischenlagerung von Produkt, beispielsweise vor der Auslieferung, oder aber von Blistern vor dem Verpouchen. Weitere Anwendungsmöglichkeiten ergeben sich als Messmethode bei Abweichungen, beispielsweise zur sicheren Charakterisierung von Bulkware in beschädigten Großgebinden. Ihre Genauigkeit, Schnelligkeit, Zerstörungsfreiheit und Mobilität machen die Methode at-line, on-line und in manchen Fällen sogar in-line einsetzbar. Dies eröffnet signifikante Qualitäts-, Effizienzsteigerung und Kostenreduktion.

Das vorschlagsgemäße Verfahren erfordert keinerlei Vorbereitung bzw. Eingriff an der Probe 2 selbst. Dies eröffnet die Entwicklung einer sehr anwenderfreundlichen Messapparatur nach dem Konzept "Schleuse auf - Probe 2 rein - Schleuse zu - messen". Die somit drastisch verkürzte Offenlagerungszeit der Probe 2 verbunden mit einfacher Handhabung ist ein entscheidender Vorteil gegenüber etablierten Methoden hin zu deutlich geringerer Fehleranfälligkeit und einer noch höheren Genauigkeit als in bisherigen Testmessungen.

Das vorschlagsgemäße Verfahren erfordert keine Hitzebelastung der Probe; dadurch wird eine Reduktion potenzieller Fehlerquellen sowie Wiederverwendbarkeit der Probe für Vergleichsmessungen ermöglicht.

Das vorschlagsgemäße Verfahren erfordert keine Verbrauchsmaterialien, was im Vergleich zu anderen Verfahren zu einer Kostenminimierung, inkl. entfallende Entsorgungskosten für umweltgefährdende Abfälle führt.

In einem weiteren Ausführungsbeispiel wird die Methode auch an Mehrkomponentensystemen, beispielsweise bei Mehrschicht-Tabletten angewandt. Dazu kann beispielsweise der Oberflächenwiderstand wahlweise an der am schnellsten sorbierenden und/oder an der feuchteempfindlichsten und/oder an der signalstärksten Komponente gemessen werden. Aus der Kenntnis der Sorptionskapazitäten und - kinetiken der einzelnen Komponenten kann auf den Feuchtegehalt bzw. Wassergehalt W der nicht gemessenen Komponenten geschlossen werden.

Alternativ oder zusätzlich wird an einer oder mehreren Komponenten / Proben 2 sowohl der Oberflächenwiderstand als auch der Volumenwiderstand gemessen. Aus dem Vergleich der beiden Messungen wird auf den Feuchtegehalt / Wassergehalt W des Gesamtsystems geschlossen.

In einem weiteren Aspekt erfolgen Feuchtegehaltsmessungen an Tablettencoatings. Mit den Messungen speziell an Coatings kann der Aufwand für das vorschlagsgemäße Verfahren reduziert werden. Die Tatsache, dass Feuchtegehaltsmessungen auch an Coatings selbst durchführbar sind, eröffnet die Möglichkeit, bereits durch eine Messung des Oberflächenwiderstandes in der Coating-Schicht getrennt von der übrigen Probe die Probenfeuchte zu messen. Bei einer Probe 2 im thermischen Gleichgewicht reflektiert die Oberflächenmessung gleichzeitig die Eigenschaft d.h. den Feuchtegehalt der ganzen Probe 2.

Die vorliegende Erfindung ermöglicht vorzugsweise "Plattform-Lösungen" unter Bereitstellung einer vereinfachten Apparatur für alle Produkte (Proben 2) mit einem bestimmten Coating. Die Folgen sind beispielsweise weniger Messbereiche, weniger Einstellaufwand, eine vereinfachtes Messverfahren, Optimierung der Ströme und Ausgangsspannungen auf den geeignetsten Messbereich, einfachere Methodenentwicklung, Reduktion der Fehlerquellen bei der Bedienung, mehr Sicherheit und damit eine Kostenreduktion.

Die vorliegende Erfindung ermöglicht vorzugsweise die Verwendung von Coatings als "aufgetragene Sensoren": Es könnten Coatings mit einer besonders hohen Feuchteabhängigkeit des elektrischen Widerstandes R gezielt ausgewählt und als "aufgetragene Sensoren" bzw. Widerstandseinrichtung 41 zur Messung eingesetzt werden. Es kann davon ausgegangen werden, dass mittels des Widerstands R rückgeschlossen werden kann auf die Feuchtigkeit bzw. den Wassergehalt W der gesamten Probe ", insbesondere wenn von einem thermodynamischen Gleichgewicht bzw. Gleichgewichtsfeuchte zwischen Coating und restlicher Probe 2 ausgegangen wird.

Dies ist vor allem bei Tabletten vorteilhaft, welche selbst eine eher geringe Feuchteabhängigkeit der Leitfähigkeit bzw. des Widerstands R aufweisen. Dies weitet die Anwendung der Methode aus mit allen sich daraus ergebenden Vorteilen.

Gemäß einem Aspekt der vorliegenden Erfindung wird mit dem vorschlagsgemäßen Verfahren die Feuchtigkeit / der Wassergehalt einer Kapsel und eines Kapselinhalts getrennt voneinander, vorzugsweise jeweils mit dem vorschlagsgemäßen Verfahren bzw. der vorschlagsgemäßen Messkammer 1, ermittelt. Da Kapselmaterialien im Vergleich zu Formulierungen teilweise erhebliche Wassermengen aufnehmen können, spielt der Wassergehalt von Kapselmaterialien eine besondere Rolle. Eine möglichst genaue Messung dieser Größe ist insbesondere vor dem Zeitpunkt der Verpackung besonders hilfreich. Das vorschlagsgemäße Verfahren ist anwendbar insbesondere auf HPMC-, Hart- und Weichgelatinekapseln, polymerbasierte Kapselmaterialien.

Die elektrische Leitfähigkeit in einem Schüttgut ist u.a. gegeben durch die Zahl der Strompfade sowie durch die einzelnen Kontaktwiderstände zwischen den hintereinander bzw. parallel vom Strom durchflossenen Partikeln. Deshalb ist speziell bei Schüttgütern eine - zumindest statistische - Kontrolle der Kontaktflächen, z.B. über die Materialdichte hilfreich für aussagekräftige und reproduzierbare Messungen. Vorteile werden hierbei dadurch erreicht, dass die Proben 2 mit definiertem Druck beaufschlagt werden. Dazu werden lose Proben (z.B. Pellets, Granulat, Pulver, Kompaktat) zur Leitfähigkeitsmessung in eine Fassung aus hoch isolierendem Kunststoff eingebracht und mit einem Stempel bei einem definierten Druck verdichtet. Um das Material nicht zu verändern, wird ein Druck unterhalb des (bzw. eines typischen) Tablettierdrucks gewählt. Es entsteht eine kompaktierte Probe, welche eine viel besser definierte Dichte aufweist als das zugehörige lose Material. Dadurch können Leitfähigkeitsmessungen mit einem sehr guten Signal-RauschVerhältnis bzw. geringen Unsicherheitsfaktor und guter Reproduzierbarkeit durchgeführt werden. Vorzugsweise werden zur Vereinfachung sowie zur Optimierung der Messung (optimale Kontaktfläche) die Druckstempel als elektrische Kontakte genutzt.

In einer weiteren, bevorzugten Ausführungsform wird zunächst die Druckabhängigkeit des elektrischen Widerstands R bei konstantem Feuchtegehalt / Wassergehalt W der Probe 2 bestimmt. Dies folgt der zugrunde liegenden Idee, dass vielfach ein so genanntes "Druckplateau" gefunden werden kann, bei welchem der elektrische Widerstand R über einen gewissen Druckbereich (nahezu) konstant bleibt oder sich weniger ändert als in anderen Abschnitten, sich also Strompfade und Kontaktflächen nicht wesentlich ändern. Die spätere Messung der Feuchteabhängigkeit des elektrischen Widerstands R erfolgt dann bei einem konstanten Druckwert auf dem Druckplateau, vorzugsweise bei einem Druckwert in der Mitte des Plateaus. Dies verringert Störeinflüsse und damit Fehlerquellen durch die Druckabhängigkeit der elektrischen Leitfähigkeit. Aus der Eliminierung der Druckabhängigkeit der Leitfähigkeit als Fehlerquelle ergeben sich Vorteile hinsichtlich der Verbesserung der Aussagekraft der Messung, d.h. der eineindeutigen Zuordnung des Widerstands R zum Feuchtgehalt / Wassergehalt W und der Verbesserung der Genauigkeit der Messung.

Ein auch unabhängig realisierbarer Aspekt der vorliegenden Erfindung betrifft die Bestimmung der Feuchte bzw. des Wassergehalts von Verpackungsmaterialien, insbesondere Packmitteln wie Folien und/oder Trockenmitteln, letztere insbesondere um die Vorbeladung der Trockenmittel mit Feuchtigkeit zu ermitteln.

Speziell bei Packmitteln, insbesondere bei polymerbasierten, können elektrischer Feldlinienverlauf in der Probe 2 sowie elektrostatische Streufelder Messungen beeinflussen. Vorzugsweise wird daher, insbesondere bei Packmitteln, mit Hilfe einer punktsymmetrischen Corbino-Geometrie gemessen. Dadurch wird ein sehr homogenes elektrisches Feld in der Probe realisiert und gleichzeitig der Einfluss von Streufeldern minimiert. Die Corbino-Geometrie kann jedoch auch an anderen Proben eingesetzt werden.

Bei der Corbino-Geometrie handelt es sich vorzugsweise eine scheibenartige, bevorzugt zumindest im Wesentlichen runde, flache oder plattenartige bzw. folienartige Probe. Von einer Mittenelektrode in der Scheibenmitte oder im Schwerpunkt zu einer vorzugsweise ringförmigen oder umlaufenden bzw. an einer Außenkante angeordneten Außenelektrode verläuft der elektrische Strom.

Bevorzugt ist konzentrisch zur Scheibe ist eine Spule bzw. ein Magnet angeordnet. Senkrecht hierzu verläuft ein, insbesondere zumindest im Wesentlichen, homogenes Magnetfeld. Wird entweder dieses oder der Strom durch die Scheibe an- oder abgestellt oder geändert, so wird als Folge des Hall-Effekts jeweils ein Strom in der Spule induziert. In einer bevorzugten Variante wird jedoch nur die Scheibenanordnung ohne Magnetfeld und Spule verwendet. Auch hier haben sich bei der zuvor beschriebenen Vorgehensweise der Widerstandsmessung gut reproduzierbare Ergebnisse gezeigt.

In einer weiteren Ausführungsform kann der Oberflächenwiderstand anstatt des Volumenwiderstands gemessen werden. Dies kann von besonderem Vorteil sein bei offen liegenden Proben (Folien, Devices, Komponenten, insbesondere Trockenmittelkarten). Insbesondere aus dem Vergleich von Oberflächen- und Volumenwiderstand kann sowohl eine Oberflächenkontamination als auch die Gesamtbelastung der Probe mit Feuchte ermittelt werden. Dies ist von Vorteil bei Offenlagerung von Packmitteln z.B. in einem Produktionsprozess, insbesondere bei Maschinenstillstand.

Gemäß einem weiteren Aspekt der vorliegenden Erfindung werden Oberflächenmessungen und Volumenmessungen der elektrischen Leitfähigkeit ermittelt und vorzugsweise miteinander verglichen. Aus dem Vergleich der Messungen kann ein Tiefen-Feuchtebeladungsprofil der Probe 2 während der Feuchteexposition erstellt werden. Die Feuchtegehaltsmessung im thermodynamischen Ungleichgewicht erleichtert die Verbesserung der Qualitätssicherung und Kosteneffizienz durch Verringerung des Ausschusses.

Vorzugsweise wird die Methode für Deckfolien insbesondere für Feuchtegehaltsmessungen an stark Wasser sorbierendem Heißsiegellack (HSL) verwendet. Dies kann als Oberflächen- oder Volumenmessung oder in Kombination der beiden geschehen. Die Kontrolle des Feuchteeintrags durch den HSL ist insbesondere bei Inhalativa von Bedeutung. Dies ist von Vorteil bei Offenlagerung von Packmitteln z.B. in einem Produktionsprozess, insbesondere bei Maschinenstillstand.

In einer weiteren Variation wird insbesondere bei offen liegenden Verbundfolien diejenige Schicht gemessen, welche das höhere Signal/Rausch-Verhältnis zeigt. Mit den Sorptionskurven und den jeweiligen Aufnahmekinetiken für Wasser kann auf jede andere unter den gleichen Klimabedingungen befindliche Komponente (z.B. Packmittel, Device) geschlossen werden. Dies ist von Vorteil bei Offenlagerung von Packmitteln z.B. in einem Produktionsprozess, insbesondere bei Maschinenstillstand.

Zur Sicherstellung der Produktqualität von feuchteempfindlichen Produkten können Sorbentien (beispielsweise Trockenmittel) auch in Form von polymerbasierten, mit gemahlenem Trockenmittel dotierten Karten oder Formteilen der Verpackung zugegeben werden.

Trockenmittel bzw. Trockenmittelkarten können auf Basis von PP oder PE, dotiert mit Molekularsieb (MS), Silikagel (SG) bzw. Bentonit, vorgesehen sein, vorzugsweise jeweils in den Gewichtsanteilenzwischen 35% und 60% m/m. Komponenten einer Sekundär- bzw. Primärverpackung (z.B. Kappe eines auf dem Markt befindlichen Schwangerschaftstests) können mit einem Sorbens dotiert werden, alternativ oder zusätzlich Devicekomponenten (z.B. Gehäuse eines Inhalers oder Formteile zum Schutz eines Blisters).

Es ist bevorzugt, den Wassergehalt von Trockenmittelkarten (TMK) ebenfalls mit Hilfe der el. Leitfähigkeit zu bestimmen, beispielsweise an TMK aus PP mit MS dotiert. Beim Versuch, zum Zweck der Qualitätssicherung die Feuchte-Vorbeladung bzw. die aktuelle Restsorptionskapazität derartiger Formteile, insbesondere TMK, konventionell zu bestimmen, stößt man auf grundsätzliche physikalische Grenzen. Ausheizen ist nicht aussagekräftig, da die Gefahr besteht, mehr als nur Wasser auszuheizen bzw. das Material zu zerstören. Gravimetrische Bestimmungen scheitern in der Praxis daran, dass Sorptionsexperimente an derartigen TMK mehrere Wochen dauern würden. Außerdem würden sie lediglich eine sehr ungenaue Angabe über die Vorbeladung mit Wasser ermöglichen.

Bei den vorangehend diskutierten Komponenten spielen solche mit maximaler Dotierung die wichtigste Rolle, da damit Verpackungsgröße und Kosten minimiert werden. Dennoch kann es, z.B. aus Gründen der mechanischen Eigenschaften, der Größe eines Devices oder funktionalen Formteils nötig sein, eine solche Komponente mit geringeren Mengen als Sorbentien zu dotieren. Die eventuell geringere Empfindlichkeit der Messmethode an der Komponente selbst kann dadurch kompensiert werden, dass beispielsweise eine Folie mit sehr hoher Feuchteempfindlichkeit des el. Widerstandes (z.B. oPA) mit verarbeitet wird (z.B. als Beilaufstreifen, Abdeckfolie oder als fest verarbeiteter Teil der Devicekomponenten bzw. Verpackungen). Alternativ oder zusätzlich kann das Trägermaterial der Komponente aus einem solchen Material (z.B. oPA) hergestellt sein.

Es ist bevorzugt, in Beutel ein Beilaufstreifen aus einem mit Molekularsieb dotierten Kunststoff einzuarbeiten. Der Wassergehalt des darin enthaltenen Molekularsiebs spiegelt sehr genau den Wassergehalt des granularen Molekularsiebs wieder.

In sehr speziellen Fällen werden multifunktionelle Trockenmittelmischungen (z.B. aus MS und SG) eingesetzt. Diese Lösung ist auch anwendbar für derartige Mischungen: Dazu wird vorgeschlagen, die Sensoreinheit (dotierter Beilaufstreifen, TMK, Devicekomponente, etc.) zusammenzusetzten aus einer mit MS dotierten und parallel einer mit SG dotierten. Das eröffnet eine breitbandige Messung über den ganzen Feuchtebereich.

Das vorschlagsgemäße Messprinzip kann auf Ca basierten Trockenmittelkarten eingesetzt werden. Eine Anwendung bei Trockenmittel enthaltenden Folien bzw. bei flexiblen Trockenmittel-Folieneinlagen, welche sich dem Innenraum einer Verpackung (z.B. einer Flasche) anpassen ist ebenfalls möglich.

In manchen Fällen kann die zu messende Komponente relativ dick sein, so dass bei Feuchteexposition im Material (Probe 2) ein Feuchtegradient innerhalb des Materials entstehen kann. In solchen Fällen kann sowohl der Oberflächen- als auch Volumenwiderstand der Probe 2 gemessen werden. Dies eröffnet die Möglichkeit, Oberflächen- und Volumenkontamination zu bestimmen und den Feuchtegehalt bzw. den Feuchteverlauf / die Feuchteverteilung innerhalb der Probe 2 auch in diesen Fällen genau zu messen.

Neben pharmazeutischen Produkten und Devices zur deren Anwendung kann die Erfindung beispielsweise bei Diagnosesystemen oder Kunststoffkarten mit Reagenzien enthaltenden Komponenten, so genannten Diagnosekarten, eingesetzt werden. Solche und andere Einrichtungen sollten in einem bestimmten Feuchtekorridor gelagert bzw. eingesetzt werden, d.h. es sollten weder bestimmte untere r.F.-Werte unterschritten, noch bestimmte obere r.F.-Werte überschritten werden.

Zur Sicherstellung der Stabilität bzw. Funktionalität einer solchen Karte (Probe 2) kann die Feuchte in der Verpackungseinheit, vorzugsweise auf der Karte selbst oder in deren Verpackung mit der Leitfähigkeitsmethode unmittelbar vor der Freigabe zur Auslieferung bzw. unmittelbar zum Einsatz gemessen werden.

Dazu kann die Verpackung einen Beilaufstreifen (z.B. oPA) als integrierter Feuchtesensor bzw. Widerstandseinrichtung 41 aufweisen. Möglich ist ferner der Einsatz einer zur Widerstandsmessung geeigneten Folie (z.B. oPA) als Siegelfolie auf der Karte oder auf Teilen davon, oder aber als Aufkleber zur Realisierung der Widerstandseinrichtung 41. Alternativ oder zusätzlich bevorzugt ist die Anfertigung der Diagnosekarte selbst aus einem Kunststoff geeignet zur Messung des Feuchtegehalts über die Leitfähigkeit. In einer Variante ist das Kartenmaterial dotiert mit Trockenmittel und ggf. feuchte-vorkonditioniert. Die Messung des Feuchtegehalts des Systems erfolgt vorzugsweise durch Widerstandsmessung an der Karte selbst oder an deren Verpackung.

Eine weitere Möglichkeit stellt die Nutzung des Chips einer integrierten Diagnoseschaltung zur Feuchtegehaltsmessung über dessen Widerstand dar. Alternativ oder zusätzlich ist die Integration eines Leitfähigkeits-Feuchtesensors in den Chip bzw. auf dem Chip (z.B. Aufbringen einer Folie, z.B. OPA zwischen zwei Pins). Hierdurch kann die Gleichgewichtsfeuchte und damit der Wassergehalt der einzelnen Komponenten des gesamten Systems bestimmt werden.

In einem weiteren Aspekt wird die Methode der el. Leitfähigkeit zur Kontrolle bzw. Bestimmung der Härte, Sprödigkeit bzw. Klebrigkeit von Tabletten bzw. Kapseln (z.B. polymerbasierten Kapseln bzw. Gelatinekapseln) genutzt.

Messungen zur Kontrolle der Härte, Sprödigkeit, Klebrigkeit an Tabletten bzw. Kapseln sind relativ aufwendig und v.a. bei elastisch und plastisch verformbaren Proben 2 oft uneindeutig bzw. mit Artefakten behaftet. Darüber hinaus sind sie relativ zeitaufwendig. Bisher wird oft versucht, diese stabilitätskritischen Parameter über den Feuchtegehalt zu kontrollieren. Mit der vorliegenden Erfindung ist es möglich, zur besseren Kontrolle stabilitätskritische Parametern anhand von Feuchtevorkonditionierten Proben 2 zu messen und einen kritischen Wert bzw. Bereich bestimmter r.F. (der Atmosphäre 4 und/oder der Probe 2) zu identifizieren. An gleichermaßen konditionierten Vergleichsproben wird die el. Leitfähigkeit (Widerstand R) in Abhängigkeit der r.F. der diese umgebenden Atmosphäre 4 gemessen. Dann wird die Leitfähigkeit bzw. der Widerstand R mit dem jeweiligen stabilitätskritischen Parameter eineindeutig identifiziert. Damit kann die Leitfähigkeit dazu genutzt werden, diese Parameter indirekt zu kontrollieren.

Messungen zur Kontrolle der Dissolution bzw. Disintegration sind relativ zeitaufwendig und können v.a. innerhalb eines Produktionsprozesses nur mit einer gewissen Verzögerung durchgeführt werden. Bisher wird oft versucht, diese stabilitätskritischen Parameter über den Feuchtegehalt / Wassergehalt W zu kontrollieren. Auch in diesem Zusammenhang kann das vorschlagsgemäße Verfahren unter Ermittlung der el. Leitfähigkeit bzw. des Widerstands R verwendet werden.

Messungen zur Kontrolle des Feinpartikelanteils von Inhalationspulvern sind besonders zeitaufwendig und können v.a. innerhalb eines Produktionsprozesses nur mit einer gewissen Verzögerung durchgeführt werden. Bisher wird oft versucht, diese stabilitätskritischen Parameter über den Feuchtegehalt bzw. Wassergehalt W zu kontrollieren. Auch in diesem Zusammenhang kann das vorschlagsgemäße Verfahren unter Ermittlung der el. Leitfähigkeit verwendet werden.

Messungen zur Kontrolle der Probenqualität bzw. des Alterungszustandes von Proben 2 sind besonders aufwendig. Insbesondere ist es sehr aufwändig, den Zeitpunkt bzw. die äußeren Parameter, unter welchen sich die Probenqualität verändern könnte, zu erkennen, ohne dazu die komplette Untersuchung mit den dafür zur Verfügung stehenden Standard-Methoden durchzuführen. Dies betrifft unter den festen Formen Proben 2, wie beispielsweise Hilfsstoffe, Wirkstoffe, Zwischenprodukte, frisch hergestellte Tabletten, Kapseln, sowie nach der Tablettierung beispielsweise unter Ausgleich von lokalen Spannungen relaxierte bzw. gealterte Tabletten bzw. Kapseln. Um die Kontrollmöglichkeiten diesbezüglich zu verbessern, können mit der vorliegenden Erfindung sehr empfindlich mögliche Veränderungen in der Probenqualität frühzeitig erkannt und die Proben 2 genaueren Untersuchungen hinsichtlich stabilitätskritischer Parameter zugeführt werden. Die Stärke der Methode ist ihre sehr hohe Empfindlichkeit gegenüber lonenkonzentrationen, beispielsweise durch Anwesenheit von Verunreinigungen, Schwankungen in den Anteilen eines Gemischs, sowie in der lonenbeweglichkeit, beispielsweise unterschiedliche bzw. sich verändernde Konformationen, Kristallinität oder Wechselwirkungen verschiedener Komponenten eines Gemisches.

Es ist möglich, eine höhere lonenmobilität möglicher höherer Reaktivität, ggf. mit stabilitätsrelevanten Aspekten und/oder eine niedrigere lonenmobilität höheren Wechselwirkungen, ggf. mit stabilitätsrelevanten Aspekten z.B. für Bruchkraft, Zerfall, Dissolution, zuzuordnen.

Bei Proben/Formulierungen, welche Veränderungen in der Kalibrierung der Leitfähigkeitskurve zeigen, wird diese Kalibrierung vorzugsweise an Rückstellmustern unmittelbar vor der Messkampagne durchgeführt. Die Messungen selbst dauern pro Messpunkt wenige Minuten.

Bisher wurde das Messprinzip als Gleichstrommessung (DC) mit einer stufenförmigen Umpolung der Stromrichtung unter einer sehr niedrigen Frequenz beschrieben. In Abhängigkeit von den materialspezifischen kapazitiven Anteilen der Ladungsverteilung in den Proben 2 und in der Apparatur bzw. Messkammer 1 kann in einer Variante eine Wechselstrommessung (AC) alternativ oder zusätzlich angewendet werden. Hierdurch können die Art der zur Leitfähigkeit beitragenden Ladungsträger bzw. ihre Mobilität (z.B. Elektronen- bzw. lonenleitung, Wechselwirkungen, z.B. Kristallwasser) ermittelt werden.

Das Messprinzip wird bei einer konstanten Temperatur, vorzugsweise nahe der Raumtemperatur, z.B. bei 25°C durchgeführt. Es ist jedoch möglich, in weiteren Varianten alternativ oder zusätzlich zu Messungen bei anderen Temperaturen überzugehen, beispielsweise mehr als 30 °C, insbesondere mehr als 40 °C und/oder weniger als 80°C, vorzugsweise weniger als 70 °C oder 60 °C.

Bei Vielkomponentensystemen bzw. -Proben können sowohl Elektronen, als auch verschiedenartige Ionen zur Leitfähigkeit beitragen. Insbesondere vor dem Hintergrund der vielfältig möglichen Wechselwirkungen und deren komplexer Temperaturabhängigkeit kann die Temperaturabhängigkeit der el. Leitfähigkeit schwer vorSignaldifferenz in der Feuchteabhängigkeit der Leitfähigkeit bewirken könnte. Dies wurde exemplarisch bewusst an einer Substanz mit einer bei 25°C eher geringen Signaldifferenz durchgeführt.

In weiteren Varianten sind Messung im thermodynamischen Ungleichgewicht zur Untersuchung der Umkonditionierung einer Probe möglich. Ein thermodynamisches Ungleichgewicht setzt sofort eine Feuchte-Umkonditionierung der Probe in Gang und während der materialspezifischen Relaxationszeit tritt innerhalb der Probe ggf. ein Gradient im Feuchtegehalt auf. Eine solche Probe kann in einem Ersatzschaltbild als eine Kette hintereinander und parallel geschalteter Widerstände betrachtet werden. Ein eineindeutiger Zusammenhang zwischen Widerstand und Feuchtegehalt ist zunächst nicht mehr gegeben, da in einem Gedankenexperiment eine relaxierte Probe (ohne inneren Feuchtegradient) und eine unrelaxierte (mit innerem Feuchtegradient) theoretisch den gleichen Widerstand zeigen könnten, jedoch unterschiedlichen Feuchtegehalt aufweisen könnten.

Vorzugsweise wird, insbesondere wenn es nötig ist, im thermischen Ungleichgewicht zu messen, z.B. um den Einfluss bei einer Offenlagerung während bestimmter Prozessschritte abzubilden, eine ausreichende Probenmenge bis zur thermischen Relaxation auf den dort relevanten Wassergehalt (z.B. Bulkwarenspezifikation) konditioniert. Dann wird eine Probe 2 der Leitfähigkeitsmessung / vorschlagsgemäßen Widerstandsmessung und ein anderer Teil der Probenmenge einer präzisen Wägung bzw. Referenzmessung zugeführt.

Im Anschluss wird das abweichende Klima bzw. die abweichende r.F. der die Probe umgebenden Atmosphäre 4 erzeugt, um beispielsweise eine Offenlagerung zu simulieren. Dabei wird vorzugsweise zeitgleich sowohl der Widerstand an der einen Probe, als auch der Feuchtegehalt an der anderen Probenmenge gravimetrisch gemessen. Damit ist ein eindeutiger Zusammenhang zwischen dem thermischen Ungleichgewicht durch das abweichende Klima auf den Widerstand und dem Einfluss auf den Feuchtegehalt gegeben. Mit diesem Zusammenhang wird vorzugsweise auf den Wassergehalt bzw. die Feuchtigkeit rückgeschlossen.

Vorzugsweise wird die zeitliche Veränderung des Feuchtegehalts an einer völlig offen liegenden Probe gemessen. Damit ist der eineindeutige Zusammenhang zwischen einem Widerstandswert R und dem zugehörigen Feuchtegehaltswert in diesem Klima zu jedem Zeitpunkt gegeben. Folglich kann der Einfluss der Kontaktanordnung bzw. der Elektroden 3 vernachlässigbar sein.

Die Verfahrensweise ist insbesondere bei drohender Umkonditionierung von Proben 2, besonders bei Maschinenstillständen, vorteilhaft, da die Oberfläche besonders schnell auf Veränderungen des Feuchtegehalts reagiert.

Die vorliegende Erfindung ist generell einsetzbar zur Messung des Feuchtegehalts von Feststoffen bzw. festen Proben 2 von Arzneimittelzubereitungen und/oder Verpackungsmaterialien.

Unterschiedliche Aspekte der vorliegenden Erfindung können innerhalb des durch die Patentansprüche vorgegebenen Rahmens einzeln und in unterschiedlichen Kombinationen realisierbar und vorteilhaft sein.

**Bezugszeichenliste:**

| | | | |
|---|---|---|---|
| 1 | Messkammer | 29 | Display |
| 2 | Arzneimittelzubereitung/Probe | 30 | Haltbarkeitsbestimmungseinrichtung |
| 3 | Elektroden | | |
| 4 | Atmosphäre | 31 | Kontaktoberfläche |
| 5 | Führungsteil | 32 | Elektrodenanordnung |
| 6 | Führung | 33 | Isolator |
| 7 | Spannmittel | 34 | Kavität |
| 8 | Gegenlager | 35 | Überwurf |
| 9 | Gewinde | 36 | Kontaktstift |
| 10 | Gegengewinde | 37 | Kammerwand |
| 11 | Anschlag | 38 | Verpackungssystem |
| 12 | Kraftmesser | 39 | Trockenmittel |
| 13 | Konditionierungsmittel | 40 | Verpackung |
| 14 | Halteeinrichtung | 41 | Widerstandseinrichtung |
| 15 | Dichtung | | |
| 16 | Trennwand | A | Knoten der ersten Elektrode |
| 17 | Kabel | B | Knoten der zweiten Elektrode |
| 18 | Messeinrichtung | C | Ausgangsknoten des Operationsverstärkers |
| 19 | Operationsverstärker | | |
| 20 | Rückkopplungswiderstand | D | Ausgangsknoten der Messeinrichtung |
| 21 | Spannungsquelle | | |
| 22 | Analog-Digital-Wandler | E | Ausgang der Feuchtebestimmungseinrichtung |
| 23 | Feuchtebestimmungseinrichtung | | |
| 24 | Zuordnungsmittel | F | Ausgang der Haltbarkeitsbestimmungseinrichtung |
| 25 | Zuordnungstabelle | | |
| 26 | Zuordnungsfunktion | R | Widerstand |
| 27 | erster Wert | W | Wassergehalt |
| 28 | zweiter Wert | | |

## Patentansprüche

1. Verfahren zur Bestimmung eines Wassergehalts (W) einer Probe in Form einer bevorzugt festen Arzneimittelzubereitung (2) und/oder eines Verpackungsmaterials, wobei in einer Messkammer (1) mindestens zwei Elektroden (3) derart in unmittelbaren Kontakt mit der Probe gebracht werden, dass die Elektroden (3) über die Probe elektrisch miteinander verbunden sind, wobei ein Widerstand (R) der Probe mittels der Elektroden (3) bestimmt und mit dem Widerstand (R) ein Wassergehalt (W) der Probe bestimmt wird, wobei die Probe innerhalb der Messkammer (1) mit einer vorgegebenen Andruckkraft der Elektroden (3) auf die Probe mit den Elektroden (3) in unmittelbaren Kontakt gebracht wird, und wobei ein Wassergehalt (W) oder eine relative Feuchtigkeit (rF) einer die Probe umgebenden Atmosphäre (4) in der Messkammer (1) eingestellt oder vorgegeben wird und die Temperatur der Atmosphäre (4) geregelt wird.

2. Verfahren gemäß Anspruch 1, **dadurch gekennzeichnet, dass** zur Bestimmung des Wassergehalts (W) ein Zuordnungsmittel (24) verwendet wird, insbesondere eine Zuordnungstabelle (25) oder Zuordnungsfunktion (26), vorzugsweise wobei das Zuordnungsmittel (24) jeweils einen mit einer Referenzmessmethode bestimmten Wassergehalt (W) der Probe einem bei demselben Wassergehalt (W) der Probe ermittelten Widerstand (R) zuordnet.

3. Verfahren gemäß einem der voranstehenden Ansprüche, **dadurch gekennzeichnet, dass** für eine Kombination einer bestimmten Darreichungsform der Probe und bestimmten Elektroden (3) jeweils ein individuelles Zuordnungsmittel (24) bestimmt und/oder verwendet wird.

4. Verfahren gemäß Anspruch 2 oder 3, **dadurch gekennzeichnet, dass** durch das Zuordnungsmittel (24) ein zu dem Widerstand (R) korrespondierender Wassergehalt (W) bestimmt wird und, vorzugsweise, der Wassergehalt (W) oder eine hieraus ermittelte, insbesondere momentane, Qualität der Probe als Ergebnis ausgegeben wird.

5. Verfahren gemäß einem der voranstehenden Ansprüche, **dadurch gekennzeichnet, dass** für eine bestimmte Darreichungsform der Probe korrespondierende Elektroden (3) in der Messkammer (1) montiert, ausgetauscht und/oder mit einer Messeinrichtung (18) zur Messung des Widerstands (R) der Probe verbunden werden.

6. Verfahren gemäß einem der voranstehenden Ansprüche, **dadurch gekennzeichnet, dass** der Wassergehalt (W) oder die relative Feuchtigkeit (rF) der Atomsphäre (4) mit einem in der Messkammer (1) vorgesehenen Konditionierungsmittel (13), insbesondere Trockenmittel (39), vorgegeben wird, und/oder dass innerhalb der Messkammer (1) dieselbe Zusammensetzung und/oder Temperatur der Atmosphäre (4) verwendet wird, wie diejenige, die bei beabsichtigtem Verpacken der Probe, insbesondere in einer Dose oder einem Blister, verwendet wird.

7. Messkammer (1) zur Ermittlung eines Widerstands (R) zum Bestimmen eines Wassergehalts (W) einer Probe in Form einer Arzneimittelzubereitung (2) und/oder eines Verpackungsmaterials, wobei die Messkammer (1) mindestens zwei Elektroden (3) aufweist und dazu ausgebildet ist, die Probe aufzunehmen und derart mit den Elektroden (3) in unmittelbaren Kontakt zu bringen, dass die Elektroden (3) durch die Probe miteinander elektrisch verbunden sind, so dass über die Elektroden (3) der Widerstand (R) der Probe bestimmbar ist, wobei die Messkammer (1) luftdicht verschließbar ist, so dass die Messkammer (1) eine die Probe umgebende Atmosphäre (4) einschließt und wobei die Messkammer (1) dazu eingerichtet ist, einen Wassergehalt (W) oder eine, insbesondere relative, Feuchtigkeit der von der Messkammer (1) eingeschlossenen Atmosphäre (4) einzustellen und die Temperatur der Atmosphäre (4) zu regeln, und wobei die Elektroden (3) ein Spannmittel (7) aufweisen, mit dem die Elektroden (3) mit einer vorgebbaren und/oder reproduzierbaren Kraft an die Probe andrückbar sind.

8. Messkammer gemäß Anspruch 7, **dadurch gekennzeichnet, dass** die Elektroden (3) zu einer Form und/oder Beschaffenheit der Probe korrespondieren, insbesondere wobei Kontaktoberflächen (31) der Elektroden (3) zumindest im Wesentlichen komplementär zu einer Oberfläche der Probe gebildet sind.

9. Messkammer gemäß Anspruch 7 oder 8, **dadurch gekennzeichnet, dass** die Elektroden (3) austauschbar sind, so dass zu einer Form und/oder Beschaffenheit der Probe korrespondiere Elektroden (3) wählbar und/oder aus einem der Form der Probenoberfläche anpassungsfähigen und gleichzeitig leitfähigen Material und/oder in der Kammer (1) montierbar sind.

10. Messkammer gemäß einem der Ansprüche 7 bis 9, **dadurch gekennzeichnet, dass** die Elektroden (3) relativ zueinander bewegbar in der Messkammer (1) gehalten sind, so dass die Elektroden (3) an die zwischen den Elektroden (3) angeordnete Probe angedrückt werden können.

11. Messkammer gemäß einem der Ansprüche 7 bis 10, **dadurch gekennzeichnet, dass** die Messkammer (1) eine Messeinrichtung (18) aufweist, die zur Messung des Widerstands (R) ausgebildet und mit den Elektroden (3) elektrisch verbunden ist.

12. Messkammer gemäß einem der Ansprüche 7 bis 11, **dadurch gekennzeichnet, dass** die Messkammer (1) eine Feuchtebestimmungseinrichtung (23) zur Bestimmung und vorzugsweise Ausgabe des Wassergehalts (W) aufweist, der zu dem Widerstand (R) korrespondiert.

## Claims

1. Method for determining a water content (W) of a sample in the form of a preferably solid pharmaceutical preparation (2) and/or a packaging material, wherein in a measurement chamber (1) at least two electrodes (3) are brought into direct contact with the sample in such a way that the electrodes (3) are electrically connected to one another via the sample, wherein a resistance (R) of the sample is determined by means of the electrodes (3) and a water content (W) of the sample is determined with the resistance (R), wherein the sample within the measurement chamber (1) is brought into direct contact with the electrodes (3) with a specified pressure force of the electrodes (3) on the sample, and wherein a water content (W) or a relative humidity (rF) of an atmosphere (4) surrounding the sample in the measurement chamber (1) is set or specified and the temperature of the atmosphere (4) is controlled.

2. Method according to claim 1, **characterized in that** an allocation means (24) is used for determining the water content (W), in particular an allocation table (25) or allocation function (26), preferably wherein the allocation means (24) allocates a water content (W) of the sample determined with a reference measuring method to a resistance (R) determined at the same water content (W) of the sample, respectively.

3. Method according to one of the preceding claims, **characterized in that** for a combination of a specific presentation form of the sample and specific electrodes (3), an individual allocation means (24) is determined and/or used, respectively.

4. Method according to claim 2 or 3, **characterized in that** a water content (W) corresponding to the resistance (R) is determined by the allocation means (24) and, preferably, the water content (W) or a quality of the sample determined therefrom, in particular an instantaneous quality, is output as a result.

5. Method according to one of the preceding claims, **characterized in that** electrodes (3) corresponding to a certain presentation form of the sample are mounted in the measurement chamber (1), exchanged and/or connected to a measuring device (18) for measuring the resistance (R) of the sample.

6. Method according to one of the preceding claims, **characterized in that** the water content (W) or the relative humidity (rF) of the atmosphere (4) is specified by a conditioning means (13), in particular a desiccant (39), provided in the measurement chamber (1), and/or **in that** the same composition and/or temperature of the atmosphere (4) is used inside the measurement chamber (1) as that used when the sample is intentionally packaged, in particular in a box or blister.

7. Measurement chamber (1) for determining a resistance (R) for determining a water content (W) of a sample in the form of a pharmaceutical preparation (2) and/or a packaging material, wherein the measurement chamber (1) has at least two electrodes (3) and is designed to receive the sample and to bring it into direct contact with the electrodes (3) in such a way that the electrodes (3) are electrically connected to one another by the sample, so that the resistance (R) of the sample can be determined via the electrodes (3), wherein the measurement chamber (1) can be closed in an airtight manner so that the measurement chamber (1) encloses an atmosphere (4) surrounding the sample, and wherein the measurement chamber (1) is adapted to adjust a water content (W) or a, in particular relative, humidity of the atmosphere (4) enclosed by the measurement chamber (1) and to control the temperature of the atmosphere (4), and wherein the electrodes (3) have a tensioning means (7) with which the electrodes (3) can be pressed against the sample with a definable and/or reproducible force.

8. Measurement chamber according to claim 7, **characterized in that** the electrodes (3) correspond to a shape and/or condition of the sample, in particular wherein contact surfaces (31) of the electrodes (3) are formed at least substantially complementary to a surface of the sample.

9. Measurement chamber according to claim 7 or 8, **characterized in that** the electrodes (3) are exchangeable, so that electrodes (3) corresponding to a shape and/or condition of the sample can be selected and/or can be made of a material which is adaptable to the shape of the sample surface and at the same time conductive and/or can be mounted in the chamber (1).

10. Measurement chamber according to one of claims 7 to 9, **characterized in that** the electrodes (3) are held in the measurement chamber (1) so as to be movable relative to one another, so that the electrodes (3) can be pressed against the sample arranged between the electrodes (3).

11. Measurement chamber according to one of claims 7 to 10, **characterized in that** the measurement chamber (1) has a measuring device (18) which is designed to measure the resistance (R) and is electrically connected to the electrodes (3).

12. Measurement chamber according to any one of claims 7 to 11, **characterized in that** the measurement chamber (1) comprises a moisture determination device (23) for determining and preferably outputting the water content (W) corresponding to the resistance (R).

## Revendications

1. Procédé pour déterminer une teneur en eau (W) d'un échantillon sous la forme d'une préparation pharmaceutique (2) de préférence solide et/ou d'un matériau d'emballage, dans lequel, dans une chambre de mesure (1), au moins deux électrodes (3) sont mises en contact direct avec l'échantillon de telle sorte que les électrodes (3) sont reliées électriquement entre elles par l'intermédiaire de l'échantillon, dans lequel une résistance (R) de l'échantillon est déterminée au moyen des électrodes (3) et une teneur en eau (W) de l'échantillon est déterminée avec la résistance (R), dans lequel l'échantillon dans la chambre de mesure (1) est mis en contact direct avec les électrodes (3) avec une force de pression prédéfinie des électrodes (3) sur l'échantillon, et dans lequel une teneur en eau (W) ou une humidité relative (rF) d'une atmosphère (4) entourant l'échantillon dans la chambre de mesure (1) est ajustée ou prédéfinie et la température de l'atmosphère (4) est contrôlée.

2. Procédé selon la revendication 1, **caractérisé en ce qu'**un moyen d'attribution (24) est utilisé pour déterminer la teneur en eau (W), en particulier un tableau d'attribution (25) ou une fonction d'attribution (26), préférentiellement le moyen d'attribution (24) attribuant une teneur en eau (W) de l'échantillon déterminée avec un procédé de mesure de référence à une résistance (R) déterminée à la même teneur en eau (W) de l'échantillon.

3. Procédé selon l'une des revendications précédentes, **caractérisé en ce qu'**un moyen d'attribution individuel (24) est déterminé et/ou utilisé pour une combinaison d'une forme de présentation spécifique de l'échantillon et d'électrodes spécifiques (3).

4. Procédé selon la revendication 2 ou 3, **caractérisé en ce qu'**une teneur en eau (W) correspondant à la résistance (R) est déterminée par le moyen d'attribution (24) et, de préférence, la teneur en eau (W) ou une qualité de l'échantillon déterminée à partir de celle-ci, en particulier une qualité instantanée, est sortie comme résultat.

5. Procédé selon l'une des revendications précédentes, **caractérisé en ce que** des électrodes (3) correspondant à une certaine forme de présentation de l'échantillon, dans la chambre de mesure (1), sont montées, échangées et/ou reliées à un dispositif de mesure (18) pour mesurer la résistance (R) de l'échantillon.

6. Procédé selon l'une des revendications précédentes, **caractérisé en ce que** la teneur en eau (W) ou l'humidité relative (rH) de l'atmosphère (4) est prédéfinie par un moyen de conditionnement (13), en particulier un dessiccateur (39), prévu dans la chambre de mesure (1), et/ou que la même composition et/ou température de l'atmosphère (4) est utilisée à l'intérieur de la chambre de mesure (1) que celle utilisée lorsque l'échantillon est conditionné intentionnellement, en particulier dans une boîte ou un blister.

7. Chambre de mesure (1) pour la détermination d'une résistance (R) pour la détermination d'une teneur en eau (W) d'un échantillon sous la forme d'une préparation pharmaceutique (2) et/ou d'un matériau d'emballage, la chambre de mesure (1) présentant au moins deux électrodes (3) et étant conçue pour recevoir l'échantillon et pour l'amener en contact direct avec les électrodes (3) de telle sorte que les électrodes (3) sont reliées électriquement entre elles par l'échantillon, de sorte que la résistance (R) de l'échantillon peut être déterminée par l'intermédiaire des électrodes (3), la chambre de mesure (1) pouvant être fermée de manière étanche à l'air de sorte que la chambre de mesure (1) enferme une atmosphère (4) entourant l'échantillon, et la chambre de mesure (1) étant adaptée pour ajuster une teneur en eau (W) ou une humidité, en particulier relative, de l'atmosphère (4) enfermée par la chambre de mesure (1) et pour contrôler la température de l'atmosphère (4), et les électrodes (3) présentant un moyen de serrage (7) avec lequel les électrodes (3) peuvent être pressées contre l'échantillon avec une force prédéterminée et/ou reproductible.

8. Chambre de mesure selon la revendication 7, **caractérisée en ce que** les électrodes (3) correspondent à une forme et/ou une condition de l'échantillon, en particulier les surfaces de contact (31) des électrodes (3) étant formées au moins sensiblement de manière complémentaire à une surface de l'échantillon.

9. Chambre de mesure selon la revendication 7 ou 8, **caractérisée en ce que** les électrodes (3) sont interchangeables, de sorte que des électrodes (3) correspondant à une forme et/ou à une condition de l'échantillon peuvent être sélectionnées et/ou fabriquées dans un matériau qui est adaptable à la forme de la surface de l'échantillon et en même temps conducteur et/ou peut être monté dans la chambre (1).

10. Chambre de mesure selon l'une des revendications 7 à 9, **caractérisée en ce que** les électrodes (3) sont maintenues dans la chambre de mesure (1) de manière à être mobiles les unes par rapport aux autres, de sorte que les électrodes (3) peuvent être pressées contre l'échantillon disposé entre les électrodes (3).

11. Chambre de mesure selon l'une des revendications 7 à 10, **caractérisée en ce que** la chambre de mesure (1) comporte un dispositif de mesure (18) qui est conçu pour mesurer la résistance (R) et qui est relié électriquement aux électrodes (3).

12. Chambre de mesure selon l'une des revendications 7 à 11, **caractérisée en ce que** la chambre de mesure (1) comprend un dispositif de détermination de l'humidité (23) pour déterminer et de préférence délivrer la teneur en eau (W) correspondant à la résistance (R).
